# EUROPEAN PATENT APPLICATION

(11) **EP 4 830 720 A1**
(43) Date of publication of application: **28.10.2026**
(21) Application number: 24906499.9
(22) Date of filing: 20.12.2024
(51) Int. Cl.: A61K 39/395, A61P 35/00

(54) **PHARMACEUTICAL COMPOSITION COMPRISING BISPECIFIC ANTIBODY SPECIFICALLY BINDING DLL3 AND CD3**

(30) Priority: 22.12.2023 CN 202311782190
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LIU, Yue, Shanghai 200245 (CN); YANG, Jianjian, Shanghai 200245 (CN); TIAN, Chenmin, Shanghai 200245 (CN)
(74) Representative: Potter Clarkson
(86) International application number: PCT/CN2024/140832
(87) International publication number: WO 2025/131024

(57) **Abstract**

Provided is a pharmaceutical composition comprising a bispecific antibody specifically binding DLL3 and CD3. Specifically, provided is a pharmaceutical composition, comprising a bispecific antibody specifically binding DLL3 and CD3 and a buffering agent.

## Description

### Technical Field

The present invention belongs to the field of biotechnological pharmaceutical formulations, and specifically relates to a pharmaceutical composition comprising a bispecific antibody specifically binding to DLL3 and CD3.

### Background Art

The statements herein merely provide background information related to the present invention and may not necessarily constitute the prior art.

Small cell lung cancer (SCLC) is a relatively malignant type of lung cancer, accounting for 10%-15% of all lung cancer cases. Small cell lung cancer tumors grow rapidly and easily metastasize, with a five-year survival rate of less than 7%. The drugs for treating small cell lung cancer are rather limited and are primarily chemotherapy drugs, such as platinum/etoposide combined chemotherapy. Patients with small cell lung cancer initially respond well to chemotherapy, but are very likely to develop resistance and experience relapse. In recent years, immunotherapies such as PD-L1 antibodies and PD1 antibodies have shown some efficacy in SCLC patients, with a response rate of about 15%. At present, there are still no specific targeted therapeutic drugs developed for this disease.

DLL3 is a ligand that inhibits Notch. Under normal conditions, DLL3 is located on the Golgi apparatus, but in cancer cells (e.g., small cell lung cancer cells), DLL3 may be expressed on the cell surface and bind to Notch in a cis manner, impeding cell-to-cell binding and the endocytosis of Notch in target cells, thereby inhibiting the Notch signaling pathway and promoting tumor cell growth. DLL3 is primarily expressed in nervous system tumors or neuroendocrine tumors, including small cell lung cancer, large cell neuroendocrine cancer, gastrointestinal neuroendocrine tumors, small cell bladder cancer, glioblastoma multiforme, metastatic castration-resistant prostate cancer, melanoma, and the like, especially SCLC. More than 80% of SCLC cases have positive expression of DLL3. However, DLL3 is not expressed in normal lung tissues and paracancerous tissues. This differential expression makes DLL3 a highly promising therapeutic target for SCLC treatment.

CD3 is a homodimeric or heterodimeric antigen expressed on T cells. Functional CD3 is formed by dimerization of two of four different chains: ε, ζ, δ, and γ. CD3 dimer pairs include γ/ε, δ/ε, and ζ/ζ. CD3 binds to the T-cell receptor complex (TCR) and is essential for T cell activation. Therefore, it has been proposed to use anti-CD3 antibodies that activate T cells for cancer treatment. However, administration of anti-CD3 antibodies may trigger T cell activation and the release of related cytokines. Excessive cytokine release leads to severe cytokine release syndrome (CRS), which is a significant challenge in the clinical application of anti-CD3 antibodies.

### Summary of the Invention

The present invention provides a pharmaceutical composition comprising a bispecific antibody specifically binding to DLL3 and CD3 and the use thereof.

In one aspect, the present invention provides a pharmaceutical composition, comprising a bispecific antibody specifically binding to DLL3 and CD3 and a buffer.

In one aspect, the present invention provides a pharmaceutical composition, comprising a bispecific antibody specifically binding to DLL3 and CD3 and a buffer, wherein:
the bispecific antibody specifically binding to DLL3 and CD3 comprises a first chain having the structure as shown in Formula I and a second chain having the structure as shown in Formula II,

   Formula I: [DLL3-VL]-[Linker 1]-[CD3-VH]-[Linker 2]-[Fc1],

   Formula II: [CD3-VL]-[Linker 3]-[DLL3-VH]-[Linker 2]-[Fc2],
the DLL3-VH has a DLL3-HCDR1 comprising the amino acid sequence of SEQ ID NO: 14, a DLL3-HCDR2 comprising the amino acid sequence of SEQ ID NO: 15, and a DLL3-HCDR3 comprising the amino acid sequence of SEQ ID NO: 16; and the DLL3-VL has a DLL3-LCDR1 comprising the amino acid sequence of SEQ ID NO: 17, a DLL3-LCDR2 comprising the amino acid sequence of SEQ ID NO: 50, and a DLL3-LCDR3 comprising the amino acid sequence of SEQ ID NO: 19; and
the CD3-VH has a CD3-HCDR1 comprising the amino acid sequence of SEQ ID NO: 56, a CD3-HCDR2 comprising the amino acid sequence of SEQ ID NO: 57, and a CD3-HCDR3 comprising the amino acid sequence of SEQ ID NO: 58; and the CD3-VL has a CD3-LCDR1 comprising the amino acid sequence of SEQ ID NO: 59, a CD3-LCDR2 comprising the amino acid sequence of SEQ ID NO: 60, and a CD3-LCDR3 comprising the amino acid sequence of SEQ ID NO: 61;
wherein: the structures as shown in Formula I and Formula II are arranged from the N-terminus to the C-terminus; the Linker 1, Linker 2, and Linker 3 are the same or different peptide linkers; the Fc1 and the Fc2 are Fc region structural sequences capable of associating with each other via the Knob-into-Hole technique; and
the buffer is a histidine buffer, an acetate buffer, a citrate buffer, a succinate buffer, or a phosphate buffer.

In some embodiments, the buffer according to the foregoing is a histidine-histidine hydrochloride buffer, a histidine-histidine acetate buffer, or a citric acid-sodium citrate buffer.

In some embodiments, the buffer according to any one of the foregoing is a histidine-histidine hydrochloride buffer.

In some embodiments, provided is the pharmaceutical composition according to any one of the foregoing, wherein the pharmaceutical composition has a pH of 4.5 to 6.0; preferably, the pharmaceutical composition has a pH of 4.5 to 5.5; and more preferably, the pharmaceutical composition has a pH of 4.8 to 5.2. In some embodiments, the pharmaceutical composition has a pH of about 4.5. In some embodiments, the pharmaceutical composition has a pH of 4.5. In some embodiments, the pharmaceutical composition has a pH of about 5.5. In some embodiments, the pharmaceutical composition has a pH of 5.5. In some embodiments, the pharmaceutical composition has a pH of about 5.0. In some embodiments, the pharmaceutical composition has a pH of 5.0.

In some embodiments, the pharmaceutical composition according to any one of the foregoing has a pH of 4.5, 4.6, 4.7, 4.8, 4.9, 5.0, 5.1, 5.2, 5.3, 5.4, 5.5, 5.6, 5.7, 5.8, 5.9, or 6.0, or any range between these point values. When reference is made to a point value in the present invention, it should be understood that this point value includes an error range. This error range is due to the laboratory environment, personnel operations, instruments, methodologies, measurement errors, and other factors. In the case of pH, it should be understood that an error range is included when the measured value is about 5.0. As an example, when a formulation is measured using an industrial pH meter, "about 5.0" means 5.0 ± 0.2 (i.e., a pH of 4.8 to 5.2).

In some embodiments, provided is the pharmaceutical composition according to any one of the foregoing, wherein the bispecific antibody specifically binding to DLL3 and CD3 is at a concentration of 1 mg/mL to 250 mg/mL.

In some embodiments, provided is the pharmaceutical composition according to any one of the foregoing, wherein the bispecific antibody specifically binding to DLL3 and CD3 is at a concentration of 1 mg/mL to 150 mg/mL. In some embodiments, provided is the pharmaceutical composition according to any one of the foregoing, wherein the bispecific antibody specifically binding to DLL3 and CD3 is at a concentration of 5 mg/mL to 150 mg/mL. In some embodiments, provided is the pharmaceutical composition according to any one of the foregoing, wherein the bispecific antibody specifically binding to DLL3 and CD3 is at a concentration of 1 mg/mL to 120 mg/mL. In some embodiments, provided is the pharmaceutical composition according to any one of the foregoing, wherein the bispecific antibody specifically binding to DLL3 and CD3 is at a concentration of 1 mg/mL to 100 mg/mL. In some embodiments, provided is the pharmaceutical composition according to any one of the foregoing, wherein the bispecific antibody specifically binding to DLL3 and CD3 is at a concentration of 5 mg/mL to 100 mg/mL. In some embodiments, provided is the pharmaceutical composition according to any one of the foregoing, wherein the bispecific antibody specifically binding to DLL3 and CD3 is at a concentration of 70 mg/mL to 70 mg/mL.

In some embodiments, provided is the pharmaceutical composition according to any one of the foregoing, wherein the bispecific antibody specifically binding to DLL3 and CD3 is at a concentration of 70 mg/mL to 150 mg/mL.

In some embodiments, provided is the pharmaceutical composition according to any one of the foregoing, wherein the bispecific antibody specifically binding to DLL3 and CD3 is at a concentration of 80 mg/mL to 120 mg/mL.

In some embodiments, provided is the pharmaceutical composition according to any one of the foregoing, wherein the bispecific antibody specifically binding to DLL3 and CD3 is at a concentration of 90 mg/mL to 110 mg/mL.

In some embodiments, provided is the pharmaceutical composition according to any one of the foregoing, wherein the bispecific antibody specifically binding to DLL3 and CD3 is at a concentration of 1 mg/mL to 20 mg/mL.

In some embodiments, provided is the pharmaceutical composition according to any one of the foregoing, wherein the bispecific antibody specifically binding to DLL3 and CD3 is at a concentration of 1 mg/mL to 10 mg/mL.

In some embodiments, provided is the pharmaceutical composition according to any one of the foregoing, wherein the bispecific antibody specifically binding to DLL3 and CD3 is at a concentration of 4 mg/mL to 6 mg/mL.

In some embodiments, provided is the pharmaceutical composition according to any one of the foregoing, wherein the bispecific antibody specifically binding to DLL3 and CD3 is at a concentration of about 1 mg/mL. In some embodiments, provided is the pharmaceutical composition according to any one of the foregoing, wherein the bispecific antibody specifically binding to DLL3 and CD3 is at a concentration of 1 mg/mL. In some embodiments, provided is the pharmaceutical composition according to any one of the foregoing, wherein the bispecific antibody specifically binding to DLL3 and CD3 is at a concentration of about 5 mg/mL. In some embodiments, provided is the pharmaceutical composition according to any one of the foregoing, wherein the bispecific antibody specifically binding to DLL3 and CD3 is at a concentration of 5 mg/mL. In some embodiments, provided is the pharmaceutical composition according to any one of the foregoing, wherein the bispecific antibody specifically binding to DLL3 and CD3 is at a concentration of about 70 mg/mL. In some embodiments, provided is the pharmaceutical composition according to any one of the foregoing, wherein the bispecific antibody specifically binding to DLL3 and CD3 is at a concentration of 70 mg/mL. In some embodiments, provided is the pharmaceutical composition according to any one of the foregoing, wherein the bispecific antibody specifically binding to DLL3 and CD3 is at a concentration of about 100 mg/mL. In some embodiments, provided is the pharmaceutical composition according to any one of the foregoing, wherein the bispecific antibody specifically binding to DLL3 and CD3 is at a concentration of 100 mg/mL. In some embodiments, provided is the pharmaceutical composition according to any one of the foregoing, wherein the bispecific antibody specifically binding to DLL3 and CD3 is at a concentration of about 150 mg/mL. In some embodiments, provided is the pharmaceutical composition according to any one of the foregoing, wherein the bispecific antibody specifically binding to DLL3 and CD3 is at a concentration of 150 mg/mL.

In some embodiments, provided is the pharmaceutical composition according to any one of the foregoing, wherein the bispecific antibody specifically binding to DLL3 and CD3 is at a concentration of 1 mg/mL, 4 mg/mL, 5 mg/mL, 6 mg/mL, 10 mg/mL, 20 mg/mL, 30 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 120 mg/mL, 130 mg/mL, 140 mg/mL, 150 mg/mL, 160 mg/mL, 170 mg/mL, 180 mg/mL, 190 mg/mL, 200 mg/mL, 210 mg/mL, 220 mg/mL, 230 mg/mL, 240 mg/mL, or 250 mg/mL, or any range between these point values. In some embodiments, provided is the pharmaceutical composition according to any one of the foregoing, wherein the bispecific antibody specifically binding to DLL3 and CD3 is at a concentration of about 1 mg/mL, about 4 mg/mL, about 5 mg/mL, about 6 mg/mL, about 10 mg/mL, about 20 mg/mL, about 30 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, about 200 mg/mL, about 210 mg/mL, about 220 mg/mL, about 230 mg/mL, about 240 mg/mL, or about 250 mg/mL.

In some embodiments, provided is the pharmaceutical composition according to any one of the foregoing, wherein the pharmaceutical composition comprises a surfactant. In some embodiments, the surfactant is a nonionic surfactant. In some embodiments, the surfactant is selected from the group consisting of poloxamers (e.g., P188), polysorbates (e.g., Polysorbate 20 and Polysorbate 80), Triton, sodium dodecyl sulfonate, sodium lauryl sulfonate, sodium octyl glucoside, lauryl sulfobetaine, myristyl sulfobetaine, linoleyl sulfobetaine, stearyl sulfobetaine, lauryl sarcosine, myristyl sarcosine, linoleyl sarcosine, stearyl sarcosine, linoleyl betaine, myristyl betaine, cetyl betaine, lauramidopropyl betaine, cocamidopropyl betaine, linoleamidopropyl betaine, myristamidopropyl betaine, palmitamidopropyl betaine, isostearamidopropyl betaine, myristamidopropyl dimethylamine, palmitamidopropyl dimethylamine, isostearamidopropyl dimethylamine, sodium methyl cocoyl taurate, sodium methyl oleoyl taurate, polyethylene glycol, polypropylene glycol, copolymers of ethylene glycol and propylene glycol, and the like.

In some embodiments, the surfactant according to the foregoing is a polysorbate or a poloxamer. In some embodiments, the surfactant is a polysorbate. In some embodiments, the surfactant is Polysorbate 80 or Poloxamer 188 (P188). In some embodiments, the surfactant is Polysorbate 80.

In some embodiments, provided is the pharmaceutical composition according to any one of the foregoing, wherein the Polysorbate 80 is at a concentration of 0.01 mg/mL to 1.0 mg/mL. In some embodiments, the Polysorbate 80 is at a concentration of 0.1 mg/mL to 1.0 mg/mL. In some embodiments, the Polysorbate 80 is at a concentration of 0.2 mg/mL to 0.6 mg/mL. In some embodiments, the Polysorbate 80 is at a concentration of 0.3 mg/mL to 0.5 mg/mL. In some embodiments, the Polysorbate 80 is at a concentration of about 0.1 mg/mL. In some embodiments, the Polysorbate 80 is at a concentration of 0.1 mg/mL. In some embodiments, the Polysorbate 80 is at a concentration of about 0.4 mg/mL. In some embodiments, the Polysorbate 80 is at a concentration of 0.4 mg/mL. In some embodiments, the Polysorbate 80 is at a concentration of about 1.0 mg/mL. In some embodiments, the Polysorbate 80 is at a concentration of 1.0 mg/mL. In some embodiments, the Polysorbate 80 is at a concentration of 0.01 mg/mL, 0.05 mg/mL, 0.1 mg/mL, 0.15 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1.0 mg/mL, or any range between these point values. In some embodiments, the Polysorbate 80 is at a concentration of about 0.01 mg/mL, about 0.05 mg/mL, about 0.1 mg/mL, about 0.15 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1.0 mg/mL.

In some embodiments, provided is the pharmaceutical composition according to any one of the foregoing, wherein the Poloxamer 188 is at a concentration of 0.5 mg/mL to 5 mg/mL. In some embodiments, the Poloxamer 188 is at a concentration of 1 mg/mL to 3 mg/mL. In some embodiments, the Poloxamer 188 is at a concentration of 1.5 mg/mL to 2.5 mg/mL. In some embodiments, the Poloxamer 188 is at a concentration of 1.8 mg/mL to 2.2 mg/mL. In some embodiments, the Poloxamer 188 is at a concentration of about 2 mg/mL. In some embodiments, the Poloxamer 188 is at a concentration of 2 mg/mL. In some embodiments, the Poloxamer 188 is at a concentration of 0.5 mg/mL, 0.8 mg/mL, 0.9 mg/mL, 1 mg/mL, 1.5 mg/mL, 1.6 mg/mL, 1.7 mg/mL, 1.8 mg/mL, 1.9 mg/mL, 2 mg/mL, 2.1 mg/mL, 2.2 mg/mL, 2.3 mg/mL, 2.4 mg/mL, 2.5 mg/mL, 2.6 mg/mL, 2.8 mg/mL, 3 mg/mL, 3.5 mg/mL, 4 mg/mL, 4.5 mg/mL, or 5.0 mg/mL, or any range between these point values. In some embodiments, the Poloxamer 188 is at a concentration of about 0.5 mg/mL, about 0.8 mg/mL, about 0.9 mg/mL, about 1 mg/mL, about 1.5 mg/mL, about 1.6 mg/mL, about 1.7 mg/mL, about 1.8 mg/mL, about 1.9 mg/mL, about 2 mg/mL, about 2.1 mg/mL, about 2.2 mg/mL, about 2.3 mg/mL, about 2.4 mg/mL, about 2.5 mg/mL, about 2.6 mg/mL, about 2.8 mg/mL, about 3 mg/mL, about 3.5 mg/mL, about 4 mg/mL, about 4.5 mg/mL, or about 5.0 mg/mL.

In some embodiments, provided is the pharmaceutical composition according to any one of the foregoing, wherein the pharmaceutical composition comprises a sugar. In some embodiments, the sugar is selected from the group consisting of conventional compounds of formula (CH₂O)ₙ and derivatives thereof, including monosaccharides, disaccharides, trisaccharides, polysaccharides, sugar alcohols, reducing sugars, non-reducing sugars, and the like. The sugar may be selected from the group consisting of sucrose, trehalose, glucose, lactose, fructose, maltose, dextran, glycerol, erythritol, glycerin, arabitol, xylitol, sorbitol, mannitol, melibiose, melezitose, raffinose, manninotriose, stachyose, maltose, lactulose, maltulose, sorbitol, maltitol, lactitol, isomaltulose, and the like.

In some embodiments, the sugar according to the foregoing is sucrose, trehalose, mannitol, or sorbitol. In some embodiments, the sugar is sucrose.

In some embodiments, provided is the pharmaceutical composition according to any one of the foregoing, wherein the sugar is at a concentration of 10 mg/mL to 120 mg/mL. In some embodiments, the sugar is at a concentration of 30 mg/mL to 100 mg/mL. In some embodiments, the sugar is at a concentration of 64 mg/mL to 96 mg/mL. In some embodiments, the sugar is at a concentration of 68 mg/mL to 92 mg/mL. In some embodiments, the sugar is at a concentration of 72 mg/mL to 88 mg/mL. In some embodiments, the sugar is at a concentration of about 30 mg/mL. In some embodiments, the sugar is at a concentration of about 80 mg/mL. In some embodiments, the sugar is at a concentration of about 100 mg/mL. In some embodiments, non-limiting examples of the concentration of the sugar include 10 mg/mL, 20 mg/mL, 30 mg/mL, 35 mg/mL, 37.5 mg/mL, 40 mg/mL, 45 mg/mL, 50 mg/mL, 55 mg/mL, 60 mg/mL, 64 mg/mL, 65 mg/mL, 68 mg/mL, 70 mg/mL, 72 mg/mL, 75 mg/mL, 80 mg/mL, 85 mg/mL, 88 mg/mL, 90 mg/mL, 92 mg/mL, 95 mg/mL, 96 mg/mL, 100 mg/mL, or 120 mg/mL, and any range between these point values. In some embodiments, the sugar is at a concentration of 30 mg/mL. In some embodiments, the sugar is at a concentration of 80 mg/mL. In some embodiments, the sugar is at a concentration of 100 mg/mL. In some embodiments, non-limiting examples of the concentration of the sugar include about 10 mg/mL, about 20 mg/mL, about 30 mg/mL, about 35 mg/mL, about 37.5 mg/mL, about 40 mg/mL, about 45 mg/mL, about 50 mg/mL, about 55 mg/mL, about 60 mg/mL, about 64 mg/mL, about 65 mg/mL, about 68 mg/mL, about 70 mg/mL, about 72 mg/mL, about 75 mg/mL, about 80 mg/mL, about 85 mg/mL, about 88 mg/mL, about 90 mg/mL, about 92 mg/mL, about 95 mg/mL, about 96 mg/mL, about 100 mg/mL, or about 120 mg/mL.

In some embodiments, provided is the pharmaceutical composition according to any one of the foregoing, wherein the sucrose is at a concentration of 10 mg/mL to 120 mg/mL. In some embodiments, the sucrose is at a concentration of 30 mg/mL to 100 mg/mL. In some embodiments, the sucrose is at a concentration of 64 mg/mL to 96 mg/mL. In some embodiments, the sucrose is at a concentration of 68 mg/mL to 92 mg/mL. In some embodiments, the sucrose is at a concentration of 72 mg/mL to 88 mg/mL. In some embodiments, the sucrose is at a concentration of about 30 mg/mL. In some embodiments, the sucrose is at a concentration of about 80 mg/mL. In some embodiments, the sucrose is at a concentration of about 100 mg/mL. In some embodiments, non-limiting examples of the concentration of the sucrose include 10 mg/mL, 20 mg/mL, 30 mg/mL, 35 mg/mL, 37.5 mg/mL, 40 mg/mL, 45 mg/mL, 50 mg/mL, 55 mg/mL, 60 mg/mL, 64 mg/mL, 65 mg/mL, 68 mg/mL, 70 mg/mL, 72 mg/mL, 75 mg/mL, 80 mg/mL, 85 mg/mL, 88 mg/mL, 90 mg/mL, 92 mg/mL, 95 mg/mL, 96 mg/mL, 100 mg/mL, or 120 mg/mL, and any range between these point values. In some embodiments, the sucrose is at a concentration of 30 mg/mL. In some embodiments, the sucrose is at a concentration of 80 mg/mL. In some embodiments, the sucrose is at a concentration of 100 mg/mL. In some embodiments, non-limiting examples of the concentration of the sucrose include about 10 mg/mL, about 20 mg/mL, about 30 mg/mL, about 35 mg/mL, about 37.5 mg/mL, about 40 mg/mL, about 45 mg/mL, about 50 mg/mL, about 55 mg/mL, about 60 mg/mL, about 64 mg/mL, about 65 mg/mL, about 68 mg/mL, about 70 mg/mL, about 72 mg/mL, about 75 mg/mL, about 80 mg/mL, about 85 mg/mL, about 88 mg/mL, about 90 mg/mL, about 92 mg/mL, about 95 mg/mL, about 96 mg/mL, about 100 mg/mL, or about 120 mg/mL.

In some embodiments, provided is the pharmaceutical composition according to any one of the foregoing, wherein the buffer is at a concentration of 5 mM to 100 mM. In some embodiments, the buffer is at a concentration of 10 mM to 50 mM. In some embodiments, the buffer is at a concentration of 10 mM to 30 mM. In some embodiments, the buffer is at a concentration of 15 mM to 25 mM. In some embodiments, the buffer is at a concentration of 24 mM to 36 mM. In some embodiments, the buffer is at a concentration of 27 mM to 33 mM. In some embodiments, the buffer is at a concentration of 14 mM to 22 mM. In some embodiments, the buffer is at a concentration of 16 mM to 20 mM. In some embodiments, the buffer is at a concentration of about 30 mM. In some embodiments, the buffer is at a concentration of about 18 mM. In some embodiments, the buffer is at a concentration of 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 27 mM, 30 mM, 33 mM, 36 mM, 40 mM, 50 mM, 60 mM, 70 mM, 80 mM, 90 mM, or 100 mM, and any range between these point values. In some embodiments, the buffer is at a concentration of 30 mM. In some embodiments, the buffer is at a concentration of 18 mM. In some embodiments, the buffer is at a concentration of about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 27 mM, about 30 mM, about 33 mM, about 36 mM, about 40 mM, about 50 mM, about 60 mM, about 70 mM, about 80 mM, about 90 mM, or about 100 mM.

In some embodiments, provided is the pharmaceutical composition according to any one of the foregoing, wherein the histidine-histidine hydrochloride buffer is at a concentration of 5 mM to 100 mM. In some embodiments, the histidine-histidine hydrochloride buffer is at a concentration of 10 mM to 50 mM. In some embodiments, the histidine-histidine hydrochloride buffer is at a concentration of 10 mM to 30 mM. In some embodiments, the histidine-histidine hydrochloride buffer is at a concentration of 15 mM to 25 mM. In some embodiments, the histidine-histidine hydrochloride buffer is at a concentration of 24 mM to 36 mM. In some embodiments, the histidine-histidine hydrochloride buffer is at a concentration of 27 mM to 33 mM. In some embodiments, the histidine-histidine hydrochloride buffer is at a concentration of 14 mM to 22 mM. In some embodiments, the histidine-histidine hydrochloride buffer is at a concentration of 16 mM to 20 mM. In some embodiments, the histidine-histidine hydrochloride buffer is at a concentration of about 30 mM. In some embodiments, the histidine-histidine hydrochloride buffer is at a concentration of about 18 mM. In some embodiments, the histidine-histidine hydrochloride buffer is at a concentration of 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 27 mM, 30 mM, 33 mM, 36 mM, 40 mM, 50 mM, 60 mM, 70 mM, 80 mM, 90 mM, or 100 mM, and any range between these point values. In some embodiments, the histidine-histidine hydrochloride buffer is at a concentration of 30 mM. In some embodiments, the histidine-histidine hydrochloride buffer is at a concentration of 18 mM. In some embodiments, the histidine-histidine hydrochloride buffer is at a concentration of about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 27 mM, about 30 mM, about 33 mM, about 36 mM, about 40 mM, about 50 mM, about 60 mM, about 70 mM, about 80 mM, about 90 mM, or about 100 mM.

In some embodiments, provided is the pharmaceutical composition according to any one of the foregoing, wherein the pharmaceutical composition further comprises an excipient; preferably, the excipient is disodium ethylenediaminetetraacetate dihydrate, DTPA (diethylenetriaminepentaacetic acid), arginine hydrochloride, glycine, methionine, proline, histidine, phenylalanine, glutamic acid, aspartic acid, sodium chloride, or calcium chloride; and more preferably, the excipient is disodium ethylenediaminetetraacetate dihydrate. In the present invention, disodium ethylenediaminetetraacetate or disodium ethylenediaminetetraacetate dihydrate comprises two molecules of water, abbreviated EDTA-2Na, with a CAS number of 6381-92-6.

In some embodiments, provided is the pharmaceutical composition according to any one of the foregoing, wherein the excipient is at a concentration of 0.01 mg/mL to 1 mg/mL; preferably, the excipient is at a concentration of 0.01 mg/mL to 0.5 mg/mL; and more preferably, the excipient is at a concentration of 0.08 mg/mL to 0.12 mg/mL. In some embodiments, the excipient is at a concentration of 0.01 mg/mL to 0.2 mg/mL. In some embodiments, the excipient is at a concentration of 0.01 mg/mL to 0.12 mg/mL. In some embodiments, the excipient is at a concentration of 0.01 mg/mL to 0.1 mg/mL. In some embodiments, the excipient is at a concentration of 0.09 mg/mL to 0.11 mg/mL. In some embodiments, the excipient is at a concentration of about 0.01 mg/mL. In some embodiments, the excipient is at a concentration of about 0.1 mg/mL. In some embodiments, the excipient is at a concentration of about 1 mg/mL. In some embodiments, the excipient is at a concentration of 0.01 mg/mL, 0.03 mg/mL, 0.05 mg/mL, 0.08 mg/mL, 0.09 mg/mL, 0.1 mg/mL, 0.11 mg/mL, 0.12 mg/mL, 0.13 mg/mL, 0.14 mg/mL, 0.15 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1 mg/mL, and any range between these point values. In some embodiments, provided is the pharmaceutical composition according to any one of the foregoing, wherein the excipient is at a concentration of 0.01 mg/mL. In some embodiments, provided is the pharmaceutical composition according to any one of the foregoing, wherein the excipient is at a concentration of 0.1 mg/mL. In some embodiments, provided is the pharmaceutical composition according to any one of the foregoing, wherein the excipient is at a concentration of 1 mg/mL. In some embodiments, the excipient is at a concentration of about 0.01 mg/mL, about 0.03 mg/mL, about 0.05 mg/mL, about 0.08 mg/mL, about 0.09 mg/mL, about 0.1 mg/mL, about 0.11 mg/mL, about 0.12 mg/mL, about 0.13 mg/mL, about 0.14 mg/mL, about 0.15 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1 mg/mL.

In some embodiments, provided is the pharmaceutical composition according to any one of the foregoing, wherein the excipient is disodium ethylenediaminetetraacetate dihydrate at 0.01 mg/mL to 1 mg/mL. In some embodiments, the excipient is disodium ethylenediaminetetraacetate dihydrate at 0.01 mg/mL to 0.5 mg/mL. In some embodiments, the excipient is disodium ethylenediaminetetraacetate dihydrate at 0.01 mg/mL to 0.2 mg/mL. In some embodiments, the excipient is disodium ethylenediaminetetraacetate dihydrate at 0.01 mg/mL to 0.12 mg/mL. In some embodiments, the excipient is disodium ethylenediaminetetraacetate dihydrate at 0.01 mg/mL to 0.1 mg/mL. In some embodiments, the excipient is disodium ethylenediaminetetraacetate dihydrate at 0.08 mg/mL to 0.12 mg/mL. In some embodiments, the excipient is disodium ethylenediaminetetraacetate dihydrate at 0.09 mg/mL to 0.11 mg/mL. In some embodiments, the excipient is disodium ethylenediaminetetraacetate dihydrate at about 0.01 mg/mL. In some embodiments, the excipient is disodium ethylenediaminetetraacetate dihydrate at about 0.1 mg/mL. In some embodiments, the excipient is disodium ethylenediaminetetraacetate dihydrate at about 1 mg/mL. In some embodiments, the disodium ethylenediaminetetraacetate is at a concentration of 0.01 mg/mL, 0.03 mg/mL, 0.05 mg/mL, 0.08 mg/mL, 0.09 mg/mL, 0.1 mg/mL, 0.11 mg/mL, 0.12 mg/mL, 0.13 mg/mL, 0.14 mg/mL, 0.15 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, or 1 mg/mL, and any range between these point values. In some embodiments, the excipient is disodium ethylenediaminetetraacetate dihydrate at 0.01 mg/mL. In some embodiments, the excipient is disodium ethylenediaminetetraacetate dihydrate at 0.1 mg/mL. In some embodiments, the excipient is disodium ethylenediaminetetraacetate dihydrate at 1 mg/mL. In some embodiments, the disodium ethylenediaminetetraacetate is at a concentration of about 0.01 mg/mL, about 0.03 mg/mL, about 0.05 mg/mL, about 0.08 mg/mL, about 0.09 mg/mL, about 0.1 mg/mL, about 0.11 mg/mL, about 0.12 mg/mL, about 0.13 mg/mL, about 0.14 mg/mL, about 0.15 mg/mL, about 0.2 mg/mL, about 0.3 mg/mL, about 0.4 mg/mL, about 0.5 mg/mL, about 0.6 mg/mL, about 0.7 mg/mL, about 0.8 mg/mL, about 0.9 mg/mL, or about 1 mg/mL.

In some embodiments, provided is the pharmaceutical composition according to any one of the foregoing, wherein in the bispecific antibody specifically binding to DLL3 and CD3,
the DLL3-VH comprises the amino acid sequence as set forth in SEQ ID NO: 33, and the DLL3-VL comprises the amino acid sequence as set forth in SEQ ID NO: 42; and
the CD3-VH comprises the amino acid sequence of SEQ ID NO: 62, and the CD3-VL comprises the amino acid sequence of SEQ ID NO: 63.

In some embodiments, provided is the pharmaceutical composition according to any one of the foregoing, wherein in the bispecific antibody specifically binding to DLL3 and CD3,
the amino acid sequence of the DLL3-VH is as set forth in SEQ ID NO: 33, and the amino acid sequence of the DLL3-VL is as set forth in SEQ ID NO: 42; and
the amino acid sequence of the CD3-VH is as set forth in SEQ ID NO: 62, and the amino acid sequence of the CD3-VL is as set forth in SEQ ID NO: 63.

In some embodiments, provided is the pharmaceutical composition according to any one of the foregoing, wherein in the bispecific antibody specifically binding to DLL3 and CD3, the Fc1 has a knob in accordance with the Knob-into-Hole technique, and the Fc2 has a hole in accordance with the Knob-into-Hole technique.

In some embodiments, provided is the pharmaceutical composition according to any one of the foregoing, wherein in the bispecific antibody specifically binding to DLL3 and CD3, the amino acid sequence of the Fc1 is as set forth in SEQ ID NO: 64; and the amino acid sequence of the Fc2 is as set forth in SEQ ID NO: 65.

In some embodiments, provided is the pharmaceutical composition according to any one of the foregoing, wherein in the bispecific antibody specifically binding to DLL3 and CD3, the amino acid sequence of the Fc1 is as set forth in SEQ ID NO: 66; and the amino acid sequence of the Fc2 is as set forth in SEQ ID NO: 67.

In some embodiments, provided is the pharmaceutical composition according to any one of the foregoing, wherein in the bispecific antibody specifically binding to DLL3 and CD3, the Linker 1, Linker 2, and Linker 3 are all peptide linkers known in the art, as long as the bispecific antibody exhibits the desired antigen binding activity. For example, the peptide linkers can be flexible peptides comprising 1-50 or 3-20 amino acid residues. In some embodiments, the peptide linkers are 1-15 amino acid residues in length. In some embodiments, the Linker 1 has the structure as shown in the general sequence formula (GGGS)nGm, wherein n is 1-5, preferably 1, 2, or 3; and m is 1-10, preferably 4, 5, 6, 7, or 8. In some embodiments, the sequence of the Linker 1 is GGGSGGGG (SEQ ID NO: 68). In some embodiments, the Linker 2 has the structure as shown in the general sequence formula Gm, wherein m is 1-10, preferably 1-5, more preferably 1, 2, or 3. In some embodiments, the sequence of the Linker 2 is G (SEQ ID NO: 69). In some embodiments, the Linker 3 has the structure as shown in the general sequence formula (GGGGS)nGm, wherein n is 1-5, preferably 1, 2, or 3; and m is 1-10, preferably 4, 5, 6, 7, or 8. In some embodiments, the sequence of the Linker 3 is GGGGSGGGG (SEQ ID NO: 70).

In some embodiments, provided is the pharmaceutical composition according to any one of the foregoing, wherein in the bispecific antibody specifically binding to DLL3 and CD3, the Linker 1, Linker 2, and Linker 3 are the same or different, and their amino acid sequences are each independently selected from the group consisting of SEQ ID NO: 68, SEQ ID NO: 69, and SEQ ID NO: 70.

In some embodiments, provided is the pharmaceutical composition according to any one of the foregoing, wherein in the bispecific antibody specifically binding to DLL3 and CD3, the amino acid sequences of the Linker 1, Linker 2, and Linker 3 are SEQ ID NO: 68, SEQ ID NO: 69, and SEQ ID NO: 70, respectively.

In some embodiments, provided is the pharmaceutical composition according to any one of the foregoing, wherein in the bispecific antibody specifically binding to DLL3 and CD3,
the amino acid sequence of Formula I, Chain 1, is as set forth in SEQ ID NO: 71, and the amino acid sequence of Formula II, Chain 2, is as set forth in SEQ ID NO: 72.

In some embodiments, provided is the pharmaceutical composition according to any one of the foregoing, wherein the pharmaceutical composition comprises the following components:
(a) 1 mg/mL to 250 mg/mL of the bispecific antibody specifically binding to DLL3 and CD3,
(b) 0.01 mg/mL to 1.0 mg/mL of Polysorbate 80 or 0.5 mg/mL to 5 mg/mL of Poloxamer 188,
(c) 10 mg/mL to 120 mg/mL of a sugar, and
(d) 5 mM to 100 mM of a buffer, the pharmaceutical composition having a pH of 4.5 to 6.0.

In some embodiments, provided is the pharmaceutical composition according to any one of the foregoing, wherein the pharmaceutical composition comprises the following components:
(a) 1 mg/mL to 250 mg/mL of the bispecific antibody specifically binding to DLL3 and CD3,
(b) 0.01 mg/mL to 1.0 mg/mL of Polysorbate 80 or 0.5 mg/mL to 5 mg/mL of Poloxamer 188,
(c) 10 mg/mL to 120 mg/mL of a sugar,
(d) 0.01 mg/mL to 1 mg/mL of disodium ethylenediaminetetraacetate dihydrate, and
(e) 5 mM to 100 mM of a buffer, the pharmaceutical composition having a pH of 4.5 to 6.0.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80 or 0.5 mg/mL to 5 mg/mL of Poloxamer 188,
(c) 30 mg/mL to 100 mg/mL of sucrose, and
(d) 10 mM to 50 mM of a histidine-histidine hydrochloride buffer, a histidine-histidine acetate buffer, or a citric acid-sodium citrate buffer, the pharmaceutical composition having a pH of 4.5 to 5.5.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80 or 0.5 mg/mL to 5 mg/mL of Poloxamer 188,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, a histidine-histidine acetate buffer, or a citric acid-sodium citrate buffer, the pharmaceutical composition having a pH of 4.5 to 5.5.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80 or 1 mg/mL to 3 mg/mL of Poloxamer 188,
(c) 30 mg/mL to 100 mg/mL of sucrose, and
(d) 10 mM to 50 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80 or 1 mg/mL to 3 mg/mL of Poloxamer 188,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 70 mg/mL to 150 mg/mL of the bispecific antibody specifically binding to DLL3 and CD3,
(b) 0.2 mg/mL to 0.6 mg/mL of Polysorbate 80 or 1 mg/mL to 3 mg/mL of Poloxamer 188,
(c) 64 mg/mL to 96 mg/mL of sucrose, and
(d) 10 mM to 30 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 70 mg/mL to 150 mg/mL of the bispecific antibody specifically binding to DLL3 and CD3,
(b) 0.2 mg/mL to 0.6 mg/mL of Polysorbate 80 or 1 mg/mL to 3 mg/mL of Poloxamer 188,
(c) 64 mg/mL to 96 mg/mL of sucrose,
(d) 0.01 mg/mL to 0.5 mg/mL of disodium ethylenediaminetetraacetate dihydrate, and
(e) 10 mM to 30 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 80 mg/mL to 120 mg/mL of the bispecific antibody specifically binding to DLL3 and CD3,
(b) 0.3 mg/mL to 0.5 mg/mL of Polysorbate 80 or 1.5 mg/mL to 2.5 mg/mL of Poloxamer 188,
(c) 64 mg/mL to 96 mg/mL of sucrose, and
(d) 15 mM to 25 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) 80 mg/mL to 120 mg/mL of the bispecific antibody specifically binding to DLL3 and CD3,
(b) 0.3 mg/mL to 0.5 mg/mL of Polysorbate 80 or 1.5 mg/mL to 2.5 mg/mL of Poloxamer 188,
(c) 64 mg/mL to 96 mg/mL of sucrose,
(d) 0.08 mg/mL to 0.12 mg/mL of disodium ethylenediaminetetraacetate dihydrate, and
(e) 15 mM to 25 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) 1 mg/mL to 250 mg/mL of the bispecific antibody specifically binding to DLL3 and CD3,
(b) 0.01 mg/mL to 1.0 mg/mL of Polysorbate 80,
(c) 10 mg/mL to 120 mg/mL of a sugar,
(d) 0.01 mg/mL to 1 mg/mL of disodium ethylenediaminetetraacetate dihydrate, and
(e) 5 mM to 100 mM of a buffer, the pharmaceutical composition having a pH of 4.5 to 6.0.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80,
(c) 30 mg/mL to 100 mg/mL of sucrose, and
(e) 10 mM to 50 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 70 mg/mL to 150 mg/mL of the bispecific antibody specifically binding to DLL3 and CD3,
(b) 0.2 mg/mL to 0.6 mg/mL of Polysorbate 80,
(c) 64 mg/mL to 96 mg/mL of sucrose,
(d) 0.01 mg/mL to 0.5 mg/mL of disodium ethylenediaminetetraacetate dihydrate, and
(e) 10 mM to 30 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 80 mg/mL to 120 mg/mL of the bispecific antibody specifically binding to DLL3 and CD3,
(b) 0.3 mg/mL to 0.5 mg/mL of Polysorbate 80,
(c) 64 mg/mL to 96 mg/mL of sucrose,
(d) 0.08 mg/mL to 0.12 mg/mL of disodium ethylenediaminetetraacetate dihydrate, and
(e) 15 mM to 25 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) 1 mg/mL to 250 mg/mL of the bispecific antibody specifically binding to DLL3 and CD3,
(b) 0.5 mg/mL to 5 mg/mL of Poloxamer 188,
(c) 10 mg/mL to 120 mg/mL of a sugar,
(d) 0.01 mg/mL to 1 mg/mL of disodium ethylenediaminetetraacetate dihydrate, and
(e) 5 mM to 100 mM of a buffer, the pharmaceutical composition having a pH of 4.5 to 6.0.

In some embodiments, provided is the pharmaceutical composition according to any one of the foregoing, wherein the pharmaceutical composition comprises the following components:
(a) 1 mg/mL to 150 mg/mL of the bispecific antibody specifically binding to DLL3 and CD3,
(b) 1 mg/mL to 3 mg/mL of Poloxamer 188,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 70 mg/mL to 150 mg/mL of the bispecific antibody specifically binding to DLL3 and CD3,
(b) 1 mg/mL to 3 mg/mL of Poloxamer 188,
(c) 64 mg/mL to 96 mg/mL of sucrose,
(d) 0.01 mg/mL to 0.5 mg/mL of disodium ethylenediaminetetraacetate dihydrate, and
(e) 10 mM to 30 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 80 mg/mL to 120 mg/mL of the bispecific antibody specifically binding to DLL3 and CD3,
(b) 1.5 mg/mL to 2.5 mg/mL of Poloxamer 188,
(c) 64 mg/mL to 96 mg/mL of sucrose,
(d) 0.08 mg/mL to 0.12 mg/mL of disodium ethylenediaminetetraacetate dihydrate, and
(e) 15 mM to 25 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 30 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 30 mM of a histidine-histidine hydrochloride buffer, the pharmaceutical composition having a pH of about 5.0.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 0.4 mg/mL of Polysorbate 80,
(c) 80 mg/mL of sucrose,
(d) 0.1 mg/mL of disodium ethylenediaminetetraacetate dihydrate, and
(e) 30 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 0.4 mg/mL of Polysorbate 80,
(c) 80 mg/mL of sucrose,
(d) 0.1 mg/mL of disodium ethylenediaminetetraacetate dihydrate, and
(e) 18 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of about 5.0.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 0.4 mg/mL of Polysorbate 80,
(c) 80 mg/mL of sucrose,
(d) 0.1 mg/mL of disodium ethylenediaminetetraacetate dihydrate, and
(e) 18 mM of a histidine-histidine hydrochloride buffer, the pharmaceutical composition having a pH of 5.0.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose, and
(d) about 30 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 0.4 mg/mL of Polysorbate 80,
(c) 80 mg/mL of sucrose, and
(d) 30 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose, and
(d) about 18 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 0.4 mg/mL of Polysorbate 80,
(c) 80 mg/mL of sucrose, and
(d) 18 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose, and
(d) about 18 mM of a histidine-histidine hydrochloride buffer, the pharmaceutical composition having a pH of about 5.0.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 0.4 mg/mL of Polysorbate 80,
(c) 80 mg/mL of sucrose, and
(d) 18 mM of a histidine-histidine hydrochloride buffer, the pharmaceutical composition having a pH of 5.0.

In some embodiments, provided is the pharmaceutical composition according to any one of the foregoing, wherein the pharmaceutical composition comprises the following components:
(a) about 100 mg/mL of the bispecific antibody specifically binding to DLL3 and CD3,
(b) about 2 mg/mL of Poloxamer 188,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of about 5.5.

In some embodiments, provided is the pharmaceutical composition according to any one of the foregoing, wherein the pharmaceutical composition comprises the following components:
(a) 100 mg/mL of the bispecific antibody specifically binding to DLL3 and CD3,
(b) 2 mg/mL of Poloxamer 188,
(c) 80 mg/mL of sucrose,
(d) 0.1 mg/mL of disodium ethylenediaminetetraacetate dihydrate, and
(e) 18 mM of a histidine-histidine hydrochloride buffer, the pharmaceutical composition having a pH of 5.5.

In some embodiments, provided is the pharmaceutical composition according to any one of the foregoing, wherein the pharmaceutical composition comprises the following components:
(a) about 70 mg/mL of the bispecific antibody specifically binding to DLL3 and CD3,
(b) about 2 mg/mL of Poloxamer 188,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of about 5.5.

In some embodiments, provided is the pharmaceutical composition according to any one of the foregoing, wherein the pharmaceutical composition comprises the following components:
(a) 70 mg/mL of the bispecific antibody specifically binding to DLL3 and CD3,
(b) 2 mg/mL of Poloxamer 188,
(c) 80 mg/mL of sucrose,
(d) 0.1 mg/mL of disodium ethylenediaminetetraacetate dihydrate, and
(e) 18 mM of a histidine-histidine hydrochloride buffer, the pharmaceutical composition having a pH of 5.5.

In some embodiments, provided is the pharmaceutical composition according to any one of the foregoing, wherein the pharmaceutical composition comprises the following components:
(a) 1 mg/mL to 20 mg/mL of the bispecific antibody specifically binding to DLL3 and CD3,
(b) 0.01 mg/mL to 1.0 mg/mL of Polysorbate 80 or 0.5 mg/mL to 5 mg/mL of Poloxamer 188,
(c) 10 mg/mL to 120 mg/mL of a sugar,
(d) 0.01 mg/mL to 1 mg/mL of disodium ethylenediaminetetraacetate dihydrate, and
(e) 5 mM to 100 mM of a buffer, the pharmaceutical composition having a pH of 4.5 to 6.0;

In some embodiments, provided is the pharmaceutical composition according to any one of the foregoing, wherein the pharmaceutical composition comprises the following components:
(a) 1 mg/mL to 10 mg/mL of the bispecific antibody specifically binding to DLL3 and CD3,
(b) 0.1 mg/mL to 1.0 mg/mL of Polysorbate 80 or 0.5 mg/mL to 5 mg/mL of Poloxamer 188,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, a histidine-histidine acetate buffer, or a citric acid-sodium citrate buffer, the pharmaceutical composition having a pH of 4.5 to 5.5.

In some embodiments, provided is the pharmaceutical composition according to any one of the foregoing, wherein the pharmaceutical composition comprises the following components:
(a) 1 mg/mL to 10 mg/mL of the bispecific antibody specifically binding to DLL3 and CD3,
(b) 0.1 mg/mL to 1.0 mg/mL of Polysorbate 80,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 4 mg/mL to 6 mg/mL of the bispecific antibody specifically binding to DLL3 and CD3,
(b) 0.2 mg/mL to 0.6 mg/mL of Polysorbate 80 or 1 mg/mL to 3 mg/mL of Poloxamer 188,
(c) 64 mg/mL to 96 mg/mL of sucrose,
(d) 0.01 mg/mL to 0.5 mg/mL of disodium ethylenediaminetetraacetate dihydrate, and
(e) 10 mM to 30 mM of a histidine-histidine hydrochloride buffer, the pharmaceutical composition having a pH of 4.5 to 5.5.

In some embodiments, provided is the pharmaceutical composition according to any one of the foregoing, wherein the pharmaceutical composition comprises the following components:
(a) 4 mg/mL to 6 mg/mL of the bispecific antibody specifically binding to DLL3 and CD3,
(b) 0.3 mg/mL to 0.5 mg/mL of Polysorbate 80,
(c) 64 mg/mL to 96 mg/mL of sucrose,
(d) 0.08 mg/mL to 0.12 mg/mL of disodium ethylenediaminetetraacetate dihydrate, and
(e) 15 mM to 25 mM of a histidine-histidine hydrochloride buffer, the pharmaceutical composition having a pH of 4.8 to 5.2.

In some embodiments, provided is the pharmaceutical composition according to any one of the foregoing, wherein the pharmaceutical composition comprises the following components:
(a) about 5 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of about 5.0.

In some embodiments, provided is the pharmaceutical composition according to any one of the foregoing, wherein the pharmaceutical composition comprises the following components:
(a) 5 mg/mL of the bispecific antibody specifically binding to DLL3 and CD3,
(b) 0.4 mg/mL of Polysorbate 80,
(c) 80 mg/mL of sucrose,
(d) 0.1 mg/mL of disodium ethylenediaminetetraacetate dihydrate, and
(e) 18 mM of a histidine-histidine hydrochloride buffer, the pharmaceutical composition having a pH of 5.0.

Those skilled in the art will appreciate that when used in reference to a range of numerical values, a cutoff value, or a particular value, "about" may mean within 1 or more than 1 standard deviation. Alternatively, "about" can mean a range that differs by up to 20% (i.e., ± 20%). Since many of the numerical values used herein are determined experimentally, those skilled in the art will appreciate that such determinations may vary from experiment to experiment and generally vary from experiment to experiment. Because of this inherent difference, it is believed that the values used herein should not be unduly limited. Thus, the term "about" is used to encompass a variation of ± 20% or less, a variation of ± 10% or less, a variation of ± 5% or less, a variation of ± 1% or less, a variation of ± 0.5% or less, or a variation of ± 0.1% or less from a specified value.

In one aspect, the present invention provides a method for preparing a lyophilized formulation, comprising the step of lyophilizing the pharmaceutical composition according to any one of the foregoing.

In one aspect, the present invention provides a lyophilized formulation obtained by the method according to the foregoing.

In one aspect, provided is the pharmaceutical composition according to any one of the foregoing provided by the present invention, which is a subcutaneous injection formulation, an intravenous injection formulation, an intraperitoneal injection formulation, or an intramuscular injection formulation. In some embodiments, provided is the pharmaceutical composition, lyophilized formulation, or reconstituted solution according to any one of the foregoing, which is a subcutaneous injection formulation.

In some embodiments, provided is the pharmaceutical composition, lyophilized formulation, or reconstituted solution according to any one of the foregoing, which is suitable for subcutaneous injection, intravenous injection, intraperitoneal injection, or intramuscular injection. In some embodiments, provided is the pharmaceutical composition, lyophilized formulation, or reconstituted solution according to any one of the foregoing, which is suitable for subcutaneous injection.

In some embodiments, provided is the pharmaceutical composition, lyophilized formulation, or reconstituted solution according to any one of the foregoing, for use in the manufacture of a medicament for subcutaneous injection, intravenous injection, intraperitoneal injection, or intramuscular injection. In some embodiments, provided is the pharmaceutical composition, lyophilized formulation, or reconstituted solution according to any one of the foregoing, for use in the manufacture of a medicament for subcutaneous injection.

The present invention also provides the pharmaceutical composition according to any one of the foregoing, or the lyophilized formulation according to any one of the foregoing, or the injectable formulation according to any one of the foregoing, for use as a medicament. In some embodiments, the medicament is used to treat a tumor or cancer.

The present invention also provides a method for treating a tumor or cancer, comprising administering to a subject a therapeutically effective amount of the pharmaceutical composition according to any one of the foregoing, or the lyophilized formulation according to any one of the foregoing, or the injectable formulation according to any one of the foregoing.

In one aspect, the present invention provides the use of the composition according to any one of the foregoing, or the lyophilized formulation according to any one of the foregoing, or the injectable formulation according to any one of the foregoing, in the manufacture of a medicament for the treatment of a tumor or cancer.

In some embodiments, the tumor or cancer according to any one of the foregoing is selected from the group consisting of: lung cancer, small cell lung cancer, large cell lung cancer, squamous cell carcinoma of the head and neck, head and neck cancer, brain cancer, glioma, glioblastoma multiforme, neuroblastoma, central nervous system cancer, neuroendocrine tumor, throat cancer, pharyngeal squamous cell carcinoma, oral squamous cell carcinoma, nasopharyngeal carcinoma, esophageal cancer, thyroid cancer, malignant pleural mesothelioma, breast cancer, liver cancer, hepatobiliary cancer, pancreatic cancer, gastric cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, large cell lung cancer, triple-negative breast cancer, and lymphoma.

In some embodiments, the tumor or cancer according to any one of the foregoing is a solid tumor.

In some embodiments, the tumor or cancer according to any one of the foregoing is lung cancer.

In some embodiments, the tumor or cancer according to any one of the foregoing is small cell lung cancer.

In some embodiments, provided is the method for treating a tumor or cancer according to any one of the foregoing, further comprising the use of a second therapeutic agent. In some embodiments, the second therapeutic agent comprises an anti-tumor agent, radiation therapy, an antibody-drug conjugate, a bispecific antibody, a bispecific antibody conjugated to an anti-tumor agent, an immune checkpoint inhibitor, or a combination thereof.

In some embodiments, provided is the method for treating a tumor or cancer according to the foregoing, wherein the second therapeutic agent is administered simultaneously, sequentially, or separately from the bispecific antibody formulation according to any one of the present invention.

The bispecific antibody formulation provided by the present invention is characterized by excellent therapeutic activity, safety, pharmacokinetic properties, and developability (e.g., stability).

### Brief Description of the Drawings

Figure 1 shows the structural schematic diagram of Format3 of the DLL3-CD3 bispecific antibodies.
Figure 2A shows the experimental results for the binding of the bispecific antibody to DLL1. The results show that the DLL3-CD3 bispecific antibody of the present invention does not bind to DLL1.
Figure 2B shows the experimental results for the binding of the bispecific antibody to DLL4. The results show that the DLL3-CD3 bispecific antibody of the present invention does not bind to DLL4.
Figure 3 shows the experimental results for the killing effects of the bispecific antibody against cells. The results show that the bispecific antibody of the present invention exhibits no killing effects against H460 cells not expressing DLL3.
Figure 4A shows the experimental results for the activation of T cells by the bispecific antibody. The results show that the bispecific antibody of the present invention exhibits significant activating effects on T cells in the presence of DLL3-expressing DLL3/H82 cells.
Figure 4B shows that the bispecific antibody of the present invention does not activate T cells in the presence of H460 negative cells not expressing DLL3.
Figure 5A shows the experimental results for the cytokine release for the bispecific antibody. The results show that PBMC cells stimulated by the bispecific antibody of the present invention secrete very low levels of IFNγ in the presence of H1184 cells.
Figure 5B shows that PBMC cells stimulated by the bispecific antibody of the present invention secrete very low levels of IL-6 in the presence of H1184 cells.

### Detailed Description of Embodiments

### Terms

The terminology used herein is for the purpose of describing embodiments only and is not intended to be limiting. Unless defined otherwise, all technical and scientific terms used herein have the same meaning as commonly understood by one of ordinary skill in the art to which the present invention belongs.

Unless the context clearly requires otherwise, throughout the specification and the claims, the words "comprising", "having", "including", and the like are to be understood in an inclusive sense, rather than an exclusive or exhaustive sense; that is to say, in the sense of "including but not limited to". Unless stated otherwise, "comprising" includes "consisting of".

The three-letter and one-letter codes of amino acids as used in the present invention are as described in J. biol. chem, 243, p3558 (1968).

The term "and/or", such as "X and/or Y", should be understood to mean "X and Y" or "X or Y", and should be used to provide explicit support for both meanings or either meaning.

As used herein, "DLL3" and "DLL3 fragment" refer to the well-known human DLL3 protein or a fragment thereof unless specified as being from a non-human species (e.g., "mouse DLL3", "mouse DLL3 fragment", "monkey DLL3", "monkey DLL3 fragment", etc.).

As used herein, "CD3" and "CD3 fragment" refer to the well-known human CD3 protein or a fragment thereof unless specified as being from a non-human species (e.g., "mouse CD3", "mouse CD3 fragment", "monkey CD3", "monkey CD3 fragment", etc.).

The term "amino acid" refers to naturally occurring and synthetic amino acids, and amino acid analogs and amino acid mimics that function in a manner similar to the naturally occurring amino acids. Naturally occurring amino acids are those encoded by genetic codes and those later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methionine methyl sulfonium. Such analogs have a modified R group (e.g., norleucine) or a modified peptide skeleton, but retain a substantially identical chemical structure to naturally occurring amino acids. Amino acid mimics refer to chemical compounds that have a structure that is different from the general chemical structure of amino acids, but function in a manner similar to naturally occurring amino acids.

The term "amino acid mutation" includes amino acid substitutions, deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antibody" is used in the broadest sense, and encompasses various antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antibody fragments, or antigen-binding portions), as long as they exhibit the desired antigen-binding activity. According to the context, skilled artisans can determine the specific meaning of "antibody".

"Native antibody" refers to naturally occurring immunoglobulin molecules. For example, native IgG antibodies are heterotetrameric glycoproteins of about 150000 daltons, composed of two light chains and two heavy chains that are disulfide-bonded. From the N-terminus to the C-terminus, each heavy chain has a variable region (VH, also known as variable heavy domain or heavy chain variable region), followed by three constant domains (CH1, CH2, and CH3). Similarly, from the N-terminus to the C-terminus, each light chain has a variable region (VL, also known as variable light domain or light chain variable domain), followed by a constant light domain (light chain constant region, CL). The terms "full-length antibody", "intact antibody", and "whole antibody" are used herein interchangeably to refer to an antibody having a structure substantially similar to a native antibody structure or having heavy chains that comprise an Fc region as defined herein.

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art. The bispecific antibodies can be classified into IgG-like bispecific antibodies and antibody-fragment-type bispecific antibodies according to the integrity of IgG molecules; the bispecific antibodies can be classified into bivalent, trivalent, tetravalent, or higher-valent bispecific antibodies according to the number of the antigen-binding regions; and the bispecific antibodies can be classified into symmetric bispecific antibodies and asymmetric bispecific antibodies according to the presence of bilateral symmetry in their structures. Among them, the bispecific antibodies based on antibody fragments, such as Fab fragments lacking Fc fragments, e.g., bispecific antibodies formed by binding two or more Fab fragments in one molecule, have low immunogenicity, small molecular weight, and high tumor tissue permeability, and typical antibody structures of this type include bispecific antibodies such as F(ab)2, scFv-Fab, and (scFv)2-Fab; and IgG-like bispecific antibodies (e.g., having Fc fragments) have relatively large molecular weight, wherein the Fc fragments facilitate later purification of the antibody and increase their solubility and stability, and the Fc portions may further bind to the receptor FcRn and increase the serum half-life of the antibody, and typical structural models of bispecific antibodies include bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); and Chen S1 et al., J Immunol Res. 2019 Feb 11; 2019: 4516041).

The amino acid sequence boundaries of CDRs may be determined by various well-known schemes, such as the "Kabat" numbering scheme (see Kabat et al. (1991), "Sequences of Proteins of Immunological Interest", 5th ed., Public Health Service, National Institutes of Health, Bethesda, MD), the "Chothia" numbering scheme, the "ABM" numbering scheme, the "Contact" numbering scheme (see Martin, ACR. Protein Sequence and Structure Analysis of Antibody Variable Domains [J]. 2001), the ImMunoGenTics (IMGT) numbering scheme (see Lefranc, M.P. et al., Dev. Comp. Immunol., 27, 55-77 (2003); and Front Immunol. 2018 Oct 16; 9: 2278), and the like. The correspondences among various numbering systems are well known to those skilled in the art. The numbering schemes of the present invention are as shown in Table 1 below.

**Table 1. Relationships among the CDR numbering systems**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless stated otherwise, the "Kabat" numbering scheme is applied to the sequences of the variable regions and CDRs in the embodiments of the present invention.

The term "antibody fragment" or "antigen-binding fragment" refers to a molecule different from an intact antibody, which comprises a portion of an intact antibody that retains the antigen-binding ability of the intact antibody. Examples of antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two subunits that are the same or different. In some embodiments, the Fc region of the human IgG heavy chain is defined as extending from the amino acid residue at position Cys226 or from Pro230 to its carboxyl terminus. Suitable native sequence Fc regions for use in the antibodies described herein include human IgG1, IgG2 (IgG2A and IgG2B), IgG3, and IgG4. Unless stated otherwise, the numbering scheme for the Fc region is the EU index.

The term "chimeric" antibody refers to an antibody in which a portion of the heavy and/or light chain is derived from a particular source or species, while the remainder of the heavy and/or light chain is derived from a different source or species.

The term "humanized" antibody refers to an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portions of the variable regions).

The term "affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1 : 1 interaction between members of a binding pair (e.g., antibody and antigen). The affinity of a molecule X for its partner Y can generally be represented by the equilibrium dissociation constant (KD). Affinity can be measured by conventional methods known in the art, including those described herein. The term "kassoc" or "ka" refers to the association rate of a particular antibody-antigen interaction, whereas the term "kdis" or "kd", as used herein, is meant to refer to the dissociation rate of a particular antibody-antigen interaction. The term "KD", as used herein, refers to the equilibrium dissociation constant, which is obtained from the ratio of kd to ka (i.e., kd/ka) and is expressed as a molar concentration (M). The KD value of an antibody can be measured using methods known in the art, such as surface plasmon resonance, ELISA, or solution equilibrium titration (SET).

The term "monoclonal antibody" refers to a population of substantially homogeneous antibodies, that is, the amino acid sequences of the antibody molecules comprised in the population are identical, except for naturally occurring mutations that may be present in minor amounts. In contrast, polyclonal antibody formulations generally comprise a plurality of different antibodies having different amino acid sequences in their variable domains, which are generally specific for different epitopes. In some embodiments, the antibody provided by the present invention is a monoclonal antibody.

The term "antigen" refers to a molecule or a portion of a molecule that is capable of being bound by a selective binding agent of an antigen-binding molecule (e.g., an antibody). An antigen may have one or more epitopes capable of interacting with different antigen-binding molecules (e.g., antibodies).

The term "epitope" refers to an area or region on an antigen that is capable of specifically binding to an antibody or an antigen-binding fragment thereof. Epitopes can be formed by contiguous amino acids (linear epitopes), or comprise non-contiguous amino acids (conformational epitopes), e.g., non-contiguous amino acids that are in spatial proximity to each other due to folding of an antigen (i.e., by tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

The term "capable of binding specifically", "binding specifically", or "binding" means that an antigen-binding molecule is capable of binding to a certain antigen or epitope with higher affinity than to other antigens or epitopes. Generally, an antibody binds to an antigen or epitope with an equilibrium dissociation constant (KD) of about 1 × 10⁻⁷ M or less (e.g., about 1 × 10⁻⁸ M or less). In some embodiments, the KD for the binding of an antibody to an antigen is 10% or less (e.g., 1%) of the KD for the binding of the antibody to a non-specific antigen (e.g., BSA or casein). KD may be measured using known methods, for example, by FACS or surface plasmon resonance assay. However, an antibody specifically binding to an antigen or an epitope thereof may have cross-reactivity to other related antigens, e.g., to corresponding antigens from other species (homologous), such as humans or monkeys, e.g., Macaca fascicularis (cynomolgus, cyno), Pan troglodytes (chimpanzee, chimp), or Callithrix jacchus (common marmoset, marmoset).

The term "no binding" means that an antigen-binding molecule is not capable of binding to a certain antigen or an epitope thereof in the specific binding manner described above, for example, when an antibody binds to an antigen or an epitope thereof with an equilibrium dissociation constant (KD) of about 1 × 10⁻⁶ M or greater.

The term "antigen-binding moiety" refers to a polypeptide molecule specifically binding to a target antigen. A specific antigen-binding moiety includes an antigen-binding domain of an antibody, e.g., comprising a heavy chain variable region and a light chain variable region. The term "antigen-binding moiety specifically binding to CD3" refers to a moiety that is capable of binding to CD3 or an epitope thereof with sufficient affinity, such that a molecule comprising the moiety can be used as a diagnostic agent and/or therapeutic agent targeting CD3. For example, the antigen-binding moiety specifically binding to CD3 has the following equilibrium dissociation constant (KD): < about 10 nM, as measured by surface plasmon resonance assay. Antigen-binding moieties include antibody fragments as defined herein, e.g., an Fab, replaced Fab, or scFv.

The term "linker" refers to a linker unit that links two polypeptide fragments. Herein, the linkers present in the same structural formula may be the same or different. The linkers may be peptide linkers comprising one or more amino acids, typically about 1-30, 2-24, or 3-15 amino acids. The linkers used herein may be the same or different. When "-" appears in a structural formula, it indicates that the units on both sides are directly linked by a covalent bond.

"Tm" is the thermal denaturation temperature (by endogenous fluorescence). When a protein is denatured (by heating or by a denaturant), the tertiary structure is opened and the aromatic amino acid microenvironment changes, resulting in a change in the emission fluorescence spectrum. In the present invention, Tm1 refers to the temperature at which the fluorescence value changes to half of the maximum value.

"Tonset" is the onset temperature of denaturation. It refers to the temperature at which a protein begins to denature, i.e., the temperature at which the fluorescence value begins to change.

"Tagg" is the onset temperature of aggregation. The temperature at which a sample begins to aggregate is monitored by detecting aggregates at two wavelengths of 266 nm and 473 nm by static light scattering. Tagg 266 refers to the onset temperature of aggregation monitored at 266 nm.

It should be understood that although specific coding nucleotide sequences are not provided in the present invention, skilled artisans can determine the corresponding coding nucleotide sequences based on amino acid sequences according to codon rules and host codon preferences.

The term "fusion" or "linkage" means that components (e.g., antigen-binding moieties and Fc domains) are covalently linked directly or via a linker.

The term "vector" means a polynucleotide molecule capable of transporting another polynucleotide linked thereto. One type of vector is the "plasmid", which refers to a circular double-stranded DNA loop into which additional DNA segments can be ligated. Another type of vector is the viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that is suitable for transformation of a host cell and comprises nucleic acid sequences that direct and/or control (in conjunction with the host cell) expression of one or more heterologous coding regions operably linked thereto. An expression construct may comprise, but is not limited to, sequences that affect or control transcription, translation, and, if introns are present, affect RNA splicing of a coding region operably linked thereto.

"Optional" or "optionally" means that the subsequently described event or circumstance may but need not occur, and that the description includes instances where the event or circumstance occurs and instances in which it does not.

The term "subject" or "individual" includes humans and non-human animals. Non-human animals include all vertebrates (e.g., mammals and non-mammals), such as non-human primates (e.g., cynomolgus monkeys), sheep, dogs, cows, chickens, amphibians, and reptiles. Unless clearly indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

When applied to an animal, human, experimental subject, cell, tissue, organ, or biological fluid, "administering" or "administration" refers to the contact of an exogenous drug, therapeutic agent, diagnostic agent, or composition with the animal, human, subject, cell, tissue, organ, or biological fluid.

The term "sample" refers to a collection of similar fluids, cells, or tissues isolated from a subject, and fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; etc.), tissue biopsy samples, fine needle punctures, surgically excised tissues, organ cultures, or cell cultures.

"Treatment" or "treat" and "treating" (and grammatical variations thereof) refers to clinical intervention intended to be applied to the treated individual, which may be performed either for prophylactic purposes or during the course of clinical pathology. Desired effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis.

The term "recurrence", "relapse", or "relapsed" refers to the return of a cancer or disease after clinical assessment of the disappearance of the disease. A diagnosis of distant cancer metastasis or local recurrence can be considered a relapse.

"Effective amount" is generally an amount sufficient to reduce the severity and/or frequency of symptoms, eliminate these symptoms and/or underlying causes, prevent the appearance of symptoms and/or their underlying causes, and/or ameliorate or improve damages caused by or associated with the disease state. In some embodiments, the effective amount is a therapeutically effective amount or a prophylactically effective amount.

"Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way.

"Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a subject.

"Buffer" refers to a buffer that resists changes in pH by the action of its acid-base conjugate components. Examples of buffers that control pH within an appropriate range include acetate, succinate, gluconate, histidine, oxalate, lactate, phosphate, citrate, tartrate, fumarate, glycylglycine, and other organic acid buffers.

"Histidine buffer" is a buffer that comprises histidine. Examples of histidine buffers include histidine-histidine hydrochloride, histidine-histidine acetate, histidine-histidine phosphate, histidine-histidine sulfate, and other buffers, preferably histidine-histidine hydrochloride buffer. The histidine-histidine hydrochloride buffer can be formulated from histidine and hydrochloric acid, or from histidine and histidine hydrochloride.

"Citrate buffer" is a buffer that comprises citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer that comprises succinate ions. Examples of succinate buffers include succinic acid-sodium succinate, succinic acid-potassium succinate, succinic acid-calcium succinate, and the like. The preferred succinate buffer is succinic acid-sodium succinate. For example, the succinic acid-sodium succinate can be formulated from succinic acid and sodium hydroxide, or from succinic acid and sodium succinate.

"Phosphate buffer" is a buffer that comprises phosphate ions. Examples of phosphate buffers include citric acid-disodium hydrogen phosphate, disodium hydrogen phosphate-sodium dihydrogen phosphate, disodium hydrogen phosphate-potassium dihydrogen phosphate, disodium hydrogen phosphate-citric acid, and the like. The preferred phosphate buffer is citric acid-disodium hydrogen phosphate.

"Acetate buffer" is a buffer that comprises acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Poloxamer" is an α-hydro-ω-hydroxypoly(oxyethylene)a-poly(oxypropylene)b-poly(oxyethylene)a block copolymer, which is formed by reacting propylene oxide with propylene glycol to form polyoxypropylene glycol, followed by adding ethylene oxide, where a is the number of oxyethylene units, and b is the number of oxypropylene units. Examples of poloxamers include, but are not limited to, Poloxamer 188 (P188 or PF68). Specifically, for Poloxamer 188, the copolymer has 75 to 85 oxyethylene units (a), 25 to 30 oxypropylene units (b), an oxyethylene (EO) content of 79.9% to 83.7%, and an average molecular weight of 7680 to 9510.

"Lyophilized formulation" refers to a formulation or pharmaceutical composition obtained after vacuum lyophilization steps from a liquid or solution form of the pharmaceutical composition or liquid or solution formulation.

The pharmaceutical composition described in the present invention can achieve a stable effect: the antibody of the pharmaceutical composition substantially retains its physical stability and/or chemical stability and/or biological activity after storage, and preferably, the pharmaceutical composition substantially retains its physical and chemical stability as well as biological activity after storage. The storage period is generally selected based on the predetermined shelf life of the pharmaceutical composition. Currently, there are a variety of analytical techniques available to measure protein stability, which can measure the stability after storage at selected temperatures over specified time periods.

A stable formulation is a formulation in which no significant changes are observed under the following conditions: storage at a refrigerated temperature (2-8°C) for at least one month, at least three months, preferably six months, more preferably one year, and even more preferably up to two years. Additionally, a stable liquid formulation includes a liquid formulation that exhibits desired characteristics after storage at temperatures including 25°C for time periods including one month, three months, or six months. It also includes a liquid formulation that exhibits desired characteristics after storage at temperatures including 40°C for time periods including four weeks, one month, three months, or six months. Typical examples of stability: generally, no more than about 10%, preferably no more than about 5% of the antibodies aggregate or degrade, as measured by SEC-HPLC. By visual analysis, the formulation is a faint yellow to nearly colorless, clear liquid, or is a colorless, clear liquid, or is clear to slightly opalescent. The concentration, pH, weight, and osmolality of the formulation exhibit a variation of no more than ± 10%, preferably a variation of no more than ± 5%. Generally, no more than about 10%, preferably no more than about 5%, of aggregates are formed in the formulation.

If the antibody shows no significant increase in aggregation, precipitation, and/or denaturation after visual inspection of color and/or clarity, or as measured by UV light scattering, size exclusion chromatography (SEC), and dynamic light scattering (DLS), then the antibody "retains its physical stability" in the pharmaceutical formulation. The changes in protein conformation can be evaluated by fluorescence spectroscopy (which determines the tertiary structure of the protein) and FTIR spectroscopy (which determines the secondary structure of the protein).

If the antibody shows no significant chemical changes, then the antibody "retains its chemical stability" in the pharmaceutical formulation. Chemical stability may be assessed by detecting and quantifying chemically altered forms of the protein. Degradation processes that commonly alter the chemical structure of proteins include hydrolysis or truncation (evaluated by methods such as size exclusion chromatography and CE-SDS), oxidation (evaluated by methods such as peptide mapping combined with mass spectrometry or MALDI/TOF/MS), deamidation (evaluated by methods such as ion-exchange chromatography, capillary isoelectric focusing, peptide mapping, and measurement of isoaspartic acid formation), and isomerization (evaluated by methods such as measurement of isoaspartic acid content and peptide mapping).

If the biological activity of the antibody at a given time is within a predetermined range of its biological activity as exhibited at the time of preparation of the pharmaceutical formulation, then the antibody "retains its biological activity" in the pharmaceutical formulation.

### Substitutional, Insertional, and Deletional Variants

In certain embodiments, provided are antigen-binding molecule variants having one or more amino acid substitutions. Substitutions may be made in the CDRs and FRs. Conservative substitutions are shown in Table 2 under the heading of "Preferred substitution". More substantial changes are provided in Table 2 under the heading of "Exemplary substitution", and as further described below with reference to amino acid side chain classes. Amino acid substitutions may be introduced into an antibody of interest and the products screened for a desired activity, for example, retained/improved antigen binding, decreased immunogenicity, or improved ADCC or CDC.

**Table 2. Amino acid substitutions**

| Original residue | Exemplary substitution | Preferred substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

Amino acids may be grouped according to common side-chain properties:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

A non-conservative substitution may refer to replacement of a member of one class with a member of another class.

One type of substitutional variants involves substitution of one or more CDR residues of a parent antibody (e.g., a humanized or human antibody). Generally, the resulting variants selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g., binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VHs or VLs are tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method to introduce diversity involves CDR-directed approaches, in which several CDR residues (e.g., 4-6 residues at a time) are randomized. CDR residues involved in antigen binding can be specifically identified, for example, using alanine scanning mutagenesis or modeling.

In certain embodiments, substitutions, insertions, or deletions may occur within one or more CDRs, as long as such changes do not substantially reduce the ability of the antibody to bind the antigen. For example, conservative changes (e.g., conservative substitutions as provided herein) that do not substantially reduce binding affinity may be made in CDRs. In certain embodiments of the variant VH and VL sequences provided above, each CDR either is unaltered, or comprises no more than one, two, or three amino acid substitutions.

A useful method for identifying residues or regions of an antibody that can be used as mutagenesis targets is called "alanine scanning mutagenesis". In this method, a residue or group of residues (e.g., charged residues such as Arg, Asp, His, Lys, and Glu) is identified and replaced with a neutral or negatively charged amino acid (e.g., Ala or polyalanine) to determine whether the interaction of the antibody with the antigen is affected. Further substitutions may be introduced at amino acid positions that show functional sensitivity to the initial substitutions. In addition, the crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include: fusions of one residue or a polypeptide with a length of 100 or more residues at the amino and/or carboxyl terminus; and intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme (or a polypeptide that increases the serum half-life of the antibody).

### Engineering of Fc Region

In one aspect, the Fc region of the bispecific antibody of the present invention comprises one or more amino acid substitutions that reduce its binding to an Fc receptor, e.g., its binding to an Fcγ receptor, and reduce or eliminate effector functions. A native IgG Fc region, specifically an IgG₁ Fc region or an IgG₄ Fc region, may cause the bispecific antibody of the present invention to target cells expressing an Fc receptor, rather than cells expressing an antigen. The engineered Fc region of the present invention exhibits reduced binding affinity for an Fc receptor and/or reduced effector functions. In some embodiments, the engineered Fc region shows no less than 50%, 80%, 90%, or 95% reduction in binding affinity for an Fc receptor as compared to the native Fc region. In some embodiments, the Fc receptor is an Fcγ receptor. In some embodiments, the Fc receptor is a human Fcγ receptor, e.g., FcγRI, FcγRIIa, FcγRIIB, or FcγRIIIa. In some embodiments, the engineered Fc region also has reduced binding affinity for a complement (e.g., C1q) as compared to the native Fc region. In some embodiments, the engineered Fc region has no reduced binding affinity for a neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, or reduced T cell priming. For the IgG1 Fc region, amino acid residue substitutions at positions such as 238, 265, 269, 270, 297, 327, and 329 can reduce effector functions. In some embodiments, the Fc region is a human IgG1 Fc region, and the amino acid residues at positions 234 and 235 are A, as numbered according to the EU index. For the IgG₄ Fc region, amino acid residue substitutions at positions such as 228 can reduce effector functions.

When the bispecific antibody comprises different binding moieties fused to the two subunits of the Fc region, it may lead to undesired homodimerization. To improve yield and purity, it will thus be advantageous to introduce modifications that promote heterodimerization in the Fc region of the bispecific antibody of the present invention. In some embodiments, the Fc region of the present invention comprises modifications in accordance with the Knob-into-Hole (KiH) technique, which method relates to introducing a knob at the interface of the first subunit and introducing a hole at the interface of the second subunit. As such, the knob can be positioned in the hole, promoting the formation of a heterodimer and inhibiting the production of a homodimer. The knob is constructed by substituting a small amino acid side chain from the interface of the first subunit with a larger side chain (e.g., tyrosine or tryptophan). The hole is created at the interface of the second subunit by substituting a large amino acid side chain with a smaller amino acid side chain (e.g., alanine or threonine). The knob and the hole are prepared by altering the nucleic acid encoding the polypeptide. Optional amino acid substitutions are as shown in the table below:

**Table 3. KiH mutation combinations**

| | | | | | | | | |
|---|---|---|---|---|---|---|---|---|
| First subunit | T36 6Y | T36 6W | T39 4W | F40 5W | T366W | T366Y F405A | T366W F405W | F405W Y407A |
| Second subunit | Y40 7T | Y40 7A | F40 5A | T39 4S | T366S L368A Y407V | T394W Y407T | T394W Y407A | T366W T394S |

In addition to the Knob-into-Hole technique, other techniques for modifying the CH3 domain of the heavy chain to achieve heterodimerization are also known in the art, e.g., WO 1996027011 A1, WO 1998050431, EP 1870459, WO 2007110205, WO 2009089004, WO 2010129304, WO 201190754, WO 2011143545, WO 2012058768, WO 2013157954, and WO 2013096291.

The C-terminus of the Fc region may be an intact C-terminus ending with amino acid residues PGK, or a truncated C-terminus, e.g., a truncated C-terminus in which one or two C-terminal amino acid residues are removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with and without a K447 residue and/or G446 + K447 residues.

### Recombination Methods

The bispecific antibody may be produced using recombination methods. For these methods, provided are one or more isolated nucleic acids encoding the bispecific antibody.

In the case of a native antibody, a native antibody fragment, or a bispecific antibody with homodimeric heavy chains, two nucleic acids are required, one for the light chain or a fragment thereof and one for the heavy chain or a fragment thereof. Such nucleic acids encode an amino acid sequence comprising the VL of the antibody and/or an amino acid sequence comprising the VH of the antibody (e.g., the light and/or heavy chain of the antibody). These nucleic acids can be on the same expression vector or on different expression vectors.

In the case of a bispecific antibody with heterodimeric heavy chains, four nucleic acids, for example, are required, one for a first light chain, one for a first heavy chain comprising a first heteromonomeric Fc-region polypeptide, one for a second light chain, and one for a second heavy chain comprising a second heteromonomeric Fc-region polypeptide. The four nucleic acids can be comprised in one or more nucleic acid molecules or expression vectors. Generally, these nucleic acids are located on two or three expression vectors; that is, one vector can comprise more than one of these nucleic acids.

In one embodiment, the present invention provides an isolated nucleic acid encoding the antibody according to the foregoing. Such nucleic acids may independently encode any one of the aforementioned polypeptide chains. In another aspect, the present invention provides one or more vectors (e.g., expression vectors) comprising such nucleic acids. In another aspect, the present invention provides a host cell comprising such nucleic acids. In one embodiment, provided is a method for preparing a bispecific antibody, wherein the method comprises culturing a host cell comprising a nucleic acid encoding the antibody, as provided above, under conditions suitable for expression of the antibody, and optionally recovering the antibody from the host cell (or host cell culture medium).

For recombinant production of a bispecific antibody, the nucleic acids encoding the protein are isolated and inserted into one or more vectors for further cloning and/or expression in a host cell. Such nucleic acids can be readily isolated and sequenced using conventional procedures (e.g., by using oligonucleotide probes that are capable of specifically binding to genes encoding the heavy and light chains of an antibody), or produced by recombination methods or obtained by chemical synthesis.

Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microbes such as filamentous fungi or yeast are suitable cloning or expression hosts for a vector encoding the antibody, including fungi and yeast strains, the glycosylation pathways of which have been "humanized", resulting in the production of an antibody with a partially or fully human glycosylation pattern. Suitable host cells for expression of (glycosylated) antibodies may also be derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures may also be used as hosts, e.g., US 5959177, US 6040498, US 6420548, US 7125978, and US 6417429. Vertebrate cells can also be used as hosts, e.g., mammalian cell lines adapted for growth in a suspension. Other examples of suitable mammalian host cell lines include an SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### Examples and Test Examples

The present invention is further described below with reference to examples and test examples, which, however, are not intended to limit the scope of the present invention. In the examples and test examples of the present invention, experimental methods for which specific conditions are not specified are generally performed under conventional conditions, such as those described in Antibodies: A Laboratory Manual and Molecular Cloning: A Laboratory Manual, Cold Spring Harbor Laboratory Press; or under conditions recommended by the manufacturers of the starting materials or commercial products. Reagents for which the specific source is not specified are conventional commercially available reagents.

### I. Preparation of Bispecific Antibodies Specifically Binding to DLL3 and CD3

### Example 1. Preparation of DLL3 Antigens, Proteins for Detection, and Stably Transfected Cell Strains

### 1.1. Construction of Cell Strains Highly Expressing DLL3

pCDH lentiviral expression vector plasmids containing SEQ ID NOs: 1-2 (synthesized by GENEWIZ) were transfected into 293T cells (Cell Bank of the Chinese Academy of Sciences, GNHu17) along with the lentiviral packaging vectors pVSVG and pCMV, using Lipofectamine 3000 (Invitrogen, L3000015) as the transfection reagent. The virus-containing culture medium supernatants were collected, filtered, and subjected to ultracentrifugation. After the supernatants were discarded, the viruses were resuspended in 0.2 mL of sterile PBS. The concentrated viruses were used to infect Chinese hamster ovary cells CHO-S (Invitrogen, R80007), DMS53 (ATCC, CRL-2062), and H82 (ATCC, HTB-175), respectively. After two to three weeks of screening with puromycin, FACS single-cell sorting was performed. The selected monoclonal cell strains were expanded and cryopreserved for subsequent experiments.

pCDH-CMV-MCS-EF1-puro lentiviral expression vector plasmids containing the LUC and GFP genes (synthesized by GENEWIZ) were transfected into 293T cells (Cell Bank of the Chinese Academy of Sciences, GNHu17) along with the lentiviral packaging vectors pVSVG and pCMV, using Lipofectamine 3000 (Invitrogen, L3000015) as the transfection reagent. The virus-containing culture medium supernatants were collected, filtered, and subjected to ultracentrifugation. After the supernatants were discarded, the viruses were resuspended in 0.2 mL of sterile PBS. The concentrated viruses were used to infect SHP77 (ATCC, CRL-2195), H1184 (ATCC, CRL-5858), and H460 (Cell Bank of the Chinese Academy of Sciences, TCHu205), respectively. After two to three weeks of screening with puromycin, FACS single-cell sorting was performed. The selected monoclonal cell strains were expanded and cryopreserved for subsequent experiments.

The sequences of the related proteins used are as follows:
1. Human DLL3 full-length protein (SEQ ID NO: 1):
2. Cynomolgus DLL3 full-length protein (SEQ ID NO: 2): 
MVSPRMSRLLSQTVILALIFIPQARPAGVFELQIHSFGPGPGPGAPRSPC
SARGPCRLFFRVCLKPGLSEEAAESPCALGAALSARGPVYTEQPEAPAPDL
PLPNGLLQVPFRDAWPGTFSLIIETWREELGDQIGGPAWSLLARVTRRRRL
AAGGPWARDIQRAGAWELRFSYRARCELPAVGTACTRLCRPRSAPSRCGP
GLRPCAPLEDECEAPPVCRAGCSLEHGFCEQPGECRCLEGWTGPLCMVPV
STSSCLGLRGPSSTTTGCLVPGPGPCDGNPCANGGSCSETPGSFECTCPRGF
YGLRCEVSGVTCADGPCFNGGLCVGGADPDSAYICHCPPGFQGSNCEKRV
DRCSLQPCRNGGLCLDLGHALRCRCRAGFAGPRCEHDLDDCAGRACANG
GTCVEGGGAHRCSCALGFGGRNCRERADPCAARPCAHGGRCYAHFSGLV
CACAPGYMGARCEFPVHPDGVSALPAAPPGLRPGDPQRYLLPPALGLLVA
AGVAGAALLLVHVRRRGHAQDAGSRLLAGTPEPSVHALPDALNNLRTQE
GPGDVPSSSVDWNRPEDVDSRGIYVISAPSIYAREVAMPLFPPLHTGRAGQ
RQNLLFPFPSSILSVK
3. Rat DLL3 full-length protein (SEQ ID NO: 3): 
MVSLQVSSLPQTLILAFLLPQALPAGVFELQIHSFGPGPGPGTPRSPCN
ARGPCRLFFRVCLKPGVSQEAAESLCALGAALSTSGPVYTEQPGVPAAALS
LPDGLVRVPFLDAWPGTFSLIIETWREQLGERAAGPAWNLLARVAGRRRL
AAGAPWARDVQRTGAWELHFSYRARCEPPAVGAACARLCRSRSAPSRCG
PGLRPCTPFPDECEAPRESLTVCRAGCSPEHGYCEEPDECHCLEGWTGPLC
TVPVSTSSCLNSRVSGPAGTGCLLPGPGPCDGNPCANGGSCSETPGSFECA
CPRGFYGPRCEVSGVTCADGPCFNGGLCVGGEDPDSAYVCHCPPAFQGSN
CERRVDRCSLQPCQNGGLCLDLGHALRCRCRAGFAGPRCEHDLDDCAGR
 
ACANGGTCVEGGGARRCSCALGFGGRDCRERADPCASRPCAHGGRCYAH
FSGLVCACAPGYMGVRCEFAVRPDGADAVPAAPRGLRQADSQRFLLPPAL
GLLAAAALAGAALLLIHVRRRGPGRDTGTRLLSGTREPSVHTLPDALNNL
RLQDGAGDGPTSSADWNHPEDGDSRSIYVIPAPSIYAREA
4. Mouse DLL3 full-length protein (SEQ ID NO: 4): 
MVSLQVSPLSQTLILAFLLPQALPAGVFELQIHSFGPGPGLGTPRSPCN
ARGPCRLFFRVCLKPGVSQEATESLCALGAALSTSVPVYTEHPGESAAALP
LPDGLVRVPFRDAWPGTFSLVIETWREQLGEHAGGPAWNLLARVVGRRR
LAAGGPWARDVQRTGTWELHFSYRARCEPPAVGAACARLCRSRSAPSRC
GPGLRPCTPFPDECEAPSVCRPGCSPEHGYCEEPDECRCLEGWTGPLCTVP
VSTSSCLNSRVPGPASTGCLLPGPGPCDGNPCANGGSCSETSGSFECACPRG
FYGLRCEVSGVTCADGPCFNGGLCVGGEDPDSAYVCHCPPGFQGSNCEKR
VDRCSLQPCQNGGLCLDLGHALRCRCRAGFAGPRCEHDLDDCAGRACAN
GGTCVEGGGSRRCSCALGFGGRDCRERADPCASRPCAHGGRCYAHFSGL
VCACAPGYMGVRCEFAVRPDGADAVPAAPRGLRQADPQRFLLPPALGLL
VAAGLAGAALLVIHVRRRGPGQDTGTRLLSGTREPSVHTLPDALNNLRLQ
DGAGDGPSSSADWNHPEDGDSRSIYVIPAPSIYAREDWLIQVLF
5. Human DLL1 full-length protein (SEQ ID NO: 5):
6. Human DLL4 full-length protein (SEQ ID NO: 6):














.

### 1. 2. Preparation of Antigens

Human DLL3 (Uniprot, Q9NYJ7), cynomolgus DLL3 (Uniprot, A0A2K5WSR4), and mouse DLL3 (Uniprot, O88516) sequences were used as templates to design human DLL3 ECD fusion proteins containing different tags, and the fusion proteins were cloned onto pTT5 vectors, respectively. After expression in 293E cells, antigens were obtained. The amino acid sequences of the related proteins are as follows:
1. His-hDLL3(ECD) (SEQ ID NO: 7): 
METDTLLLWVLLLWVPGSTGHHHHHHHHHHGGGGSGGGGSAGVF
ELQIHSFGPGPGPGAPRSPCSARLPCRLFFRVCLKPGLSEEAAESPCALGAA
LSARGPVYTEQPGAPAPDLPLPDGLLQVPFRDAWPGTFSFIIETWREELGD
QIGGPAWSLLARVAGRRRLAAGGPWARDIQRAGAWELRFSYRARCEPPA
VGTACTRLCRPRSAPSRCGPGLRPCAPLEDECEAPLVCRAGCSPEHGFCEQ
PGECRCLEGWTGPLCTVPVSTSSCLSPRGPSSATTGCLVPGPGPCDGNPCA
NGGSCSETPRSFECTCPRGFYGLRCEVSGVTCADGPCFNGGLCVGGADPDS
AYICHCPPGFQGSNCEKRVDRCSLQPCRNGGLCLDLGHALRCRCRAGFAG
PRCEHDLDDCAGRACANGGTCVEGGGAHRCSCALGFGGRDCRERADPCA
ARPCAHGGRCYAHFSGLVCACAPGYMGARCEFPVHPDGASALPAAPPGL
RPGDPQRYL
 Notes: The signal peptide sequence is indicated with a dotted line, the his tag and the linker are single underlined, and the extracellular region of DLL3 is double underlined.
2. Fc-hDLL3(ECD) (SEQ ID NO: 8): 
METDTLLLWVLLLWVPGSTGEPKSSDKTHTCPPCPAPELLGGPSVFLF
PPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQP
REPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLS
PGGGGGSGGGGSAGVFELQIHSFGPGPGPGAPRSPCSARLPCRLFFRVCLKP
GLSEEAAESPCALGAALSARGPVYTEQPGAPAPDLPLPDGLLQVPFRDAWP
GTFSFIIETWREELGDQIGGPAWSLLARVAGRRRLAAGGPWARDIQRAGA
WELRFSYRARCEPPAVGTACTRLCRPRSAPSRCGPGLRPCAPLEDECEAPL
VCRAGCSPEHGFCEQPGECRCLEGWTGPLCTVPVSTSSCLSPRGPSSATTG
CLVPGPGPCDGNPCANGGSCSETPRSFECTCPRGFYGLRCEVSGVTCADGP
CFNGGLCVGGADPDSAYICHCPPGFQGSNCEKRVDRCSLQPCRNGGLCLD
LGHALRCRCRAGFAGPRCEHDLDDCAGRACANGGTCVEGGGAHRCSCAL
GFGGRDCRERADPCAARPCAHGGRCYAHFSGLVCACAPGYMGARCEFPV
HPDGASALPAAPPGLRPGDPQRYL
 Notes: The signal peptide sequence is indicated with a dotted line, the Fc tag and the linker are single underlined, and the extracellular region of DLL3 is double underlined.
3. hDLL3(ECD)-strep twin (SEQ ID NO: 9): 
METDTLLLWVLLLWVPGSTGAGVFELQIHSFGPGPGPGAPRSPCSARL
PCRLFFRVCLKPGLSEEAAESPCALGAALSARGPVYTEQPGAPAPDLPLPD
GLLQVPFRDAWPGTFSFIIETWREELGDQIGGPAWSLLARVAGRRRLAAG
GPWARDIQRAGAWELRFSYRARCEPPAVGTACTRLCRPRSAPSRCGPGLR
PCAPLEDECEAPLVCRAGCSPEHGFCEQPGECRCLEGWTGPLCTVPVSTSS
CLSPRGPSSATTGCLVPGPGPCDGNPCANGGSCSETPRSFECTCPRGFYGLR
CEVSGVTCADGPCFNGGLCVGGADPDSAYICHCPPGFQGSNCEKRVDRCS
LQPCRNGGLCLDLGHALRCRCRAGFAGPRCEHDLDDCAGRACANGGTCV
EGGGAHRCSCALGFGGRDCRERADPCAARPCAHGGRCYAHFSGLVCACA
PGYMGARCEFPVHPDGASALPAAPPGLRPGDPQRYLWSHPQFEKGGGSGG
GSGGSAWSHPQFEK
 Notes: The signal peptide sequence is indicated with a dotted line, the extracellular region of DLL3 is double underlined, and the strep twin tag is single underlined.
4. cynoDLL3(ECD)-strep twin (SEQ ID NO: 10): 
METDTLLLWVLLLWVPGSTGAGVFELQIHSFGPGPGPGAPRSPCSARG
PCRLFFRVCLKPGLSEEAAESPCALGAALSARGPVYTEQPEAPAPDLPLPN
GLLQVPFRDAWPGTFSLIIETWREELGDQIGGPAWSLLARVTRRRRLAAGG
PWARDIQRAGAWELRFSYRARCELPAVGTACTRLCRPRSAPSRCGPGLRP
CAPLEDECEAPPVCRAGCSLEHGFCEQPGECRCLEGWTGPLCMVPVSTSSC
LGLRGPSSTTTGCLVPGPGPCDGNPCANGGSCSETPGSFECTCPRGFYGLR
CEVSGVTCADGPCFNGGLCVGGADPDSAYICHCPPGFQGSNCEKRVDRCS
LQPCRNGGLCLDLGHALRCRCRAGFAGPRCEHDLDDCAGRACANGGTCV
EGGGAHRCSCALGFGGRNCRERADPCAARPCAHGGRCYAHFSGLVCACA
PGYMGARCEFPVHPDGVSALPAAPPGLRPGDPQRYLWSHPQFEKGGGSGG
GSGGSAWSHPQFEK
 Notes: The signal peptide sequence is indicated with a dotted line, the extracellular region of DLL3 is double underlined, and the strep twin tag is single underlined.
5. mouDLL3(ECD)-strep twin (SEQ ID NO: 11):

METDTLLLWVLLLWVPGSTGAGVFELQIHSFGPGPGLGTPRSPCNAR
GPCRLFFRVCLKPGVSQEATESLCALGAALSTSVPVYTEHPGESAAALPLP
DGLVRVPFRDAWPGTFSLVIETWREQLGEHAGGPAWNLLARVVGRRRLA
AGGPWARDVQRTGTWELHFSYRARCEPPAVGAACARLCRSRSAPSRCGP
GLRPCTPFPDECEAPSVCRPGCSPEHGYCEEPDECRCLEGWTGPLCTVPVS
TSSCLNSRVPGPASTGCLLPGPGPCDGNPCANGGSCSETSGSFECACPRGFY
GLRCEVSGVTCADGPCFNGGLCVGGEDPDSAYVCHCPPGFQGSNCEKRV
DRCSLQPCQNGGLCLDLGHALRCRCRAGFAGPRCEHDLDDCAGRACANG
GTCVEGGGSRRCSCALGFGGRDCRERADPCASRPCAHGGRCYAHFSGLVC
ACAPGYMGVRCEFAVRPDGADAVPAAPRGLRQADPQRFLWSHPQFEKGG
GSGGGSGGSAWSHPQFEK

Notes: The signal peptide sequence is indicated with a dotted line, the extracellular region of DLL3 is double underlined, and the strep twin tag is single underlined.

### Example 2. Preparation of Anti-Human DLL3 Antibodies

Monoclonal antibodies against human DLL3 were prepared using the hybridoma technique. The immunization method was as follows:
For the first group, cross-immunization was performed using the adjuvants TiterMax^{®} Gold Adjuvant (Sigma Cat No. T2684) and Thermo Imject^{®} Alum (Thermo Cat No. 77161), with His-hDLL3(ECD) (SEQ ID NO: 7) as the immunogen. The ratio of the antigen to the adjuvant (TiterMax^{®} Gold Adjuvant) was 1 : 1, and the ratio of the antigen to the adjuvant (Thermo Imject^{®} Alum) was 3 : 1. The dose for the first immunization was 50 µg/mouse/immunization, and the dose for boost immunization was 25 µg/mouse/immunization. The antibody titers in the mouse sera were determined by ELISA.

The immunizing antigens for the second group of mice were DLL3 CHO-S cells and Fc-hDLL3(ECD) (SEQ ID NO: 8), and immunizations were alternately performed with cell and protein antigens. Cell immunization was performed by injecting intraperitoneally into each mouse 0.1 mL of a cell suspension diluted with normal saline to a concentration of 10⁸/mL. For the Fc-hDLL3(ECD) antigen, cross-immunization was performed using the adjuvants TiterMax^{®} Gold Adjuvant (Sigma Cat No. T2684) and Thermo Imject^{®} Alum (Thermo Cat No. 77161). The ratio of the antigen to the adjuvant (TiterMax^{®} Gold Adjuvant) was 1 : 1, and the ratio of the antigen to the adjuvant (Thermo Imject^{®} Alum) was 3 : 1. The dose for the first immunization was 50 µg/mouse/immunization, and the dose for boost immunization was 25 µg/mouse/immunization. The antibody titers in the mouse sera were determined by ELISA.

Spleen lymphocytes and the myeloma cells, Sp2/0 cells (ATCC^{®} CRL-8287^{™}), were fused following an optimized electrofusion procedure to obtain hybridoma cells. Based on the growth density of hybridoma cells, the hybridoma culture supernatants were detected using ELISA for detecting binding to DLL3 protein and FACS for detecting binding to DLL3 CHO-S cells. Clones that bind to human DLL3 protein, cynomolgus DLL3 protein, and DLL3 CHO-S cells but do not bind to wild-type CHO-S cells were selected, cryopreserved, expanded, conserved for seed stock, and subjected to one to two rounds of subcloning in time until single-cell clones were obtained. Through the screening experiment above, the hybridoma clone mAb6 was obtained.

The hybridoma clones were expanded, and RNA was extracted. Reverse transcription amplification (RT-PCR) was performed using degenerate primers of mouse Ig, and the variable region sequences of the antibody were finally obtained.
mAb6 heavy chain variable region (SEQ ID NO: 12): 
EVQLQQSGPVLVKPGASVKMSCKASGYTFTDYYMNWVKQSHGKSL
EWIGIINPYSGVTIYNHKFKGKATLTVDKSSNTAYMELNSLTSEDSAVYYC
ARQDSLLDYAMDYWGQGTSVTVSS
mAb6 light chain variable region (SEQ ID NO: 13):

DILMTQSPSSMSVSLGDTVSITCHASQGISGNMGWLQQKPGKSFKGLI
YHGANLEDGVPSRFSGSGSGADYSLTISSLESEDFADYYCVQYAQFPYTFG
GGTKLEIK

**Table 4. CDRs of the antibody**

| Antibody | Name | Sequence | SEQ ID NO |
|---|---|---|---|
| | HCDR1 | DYYMN | SEQ ID NO: 14 |
| | HCDR2 | IINPYSGVTIYNHKFKG | SEQ ID NO: 15 |
| mAb6 | HCDR3 | QDSLLDYAMDY | SEQ ID NO: 16 |
| | LCDR1 | HASQGISGNMG | SEQ ID NO: 17 |
| | LCDR2 | HGANLED | SEQ ID NO: 18 |
| | LCDR3 | VQYAQFPYT | SEQ ID NO: 19 |

| | | | |
|---|---|---|---|
| Notes: The amino acid residues of the CDRs of the VH/VL were determined and annotated using the Kabat numbering system. | | | |

The heavy chain variable region and the light chain variable region of the murine antibody were cloned into the pTT5 vector plasmids containing a human IgG1 heavy chain constant region as set forth in SEQ ID NO: 20 and a κ light chain constant region as set forth in SEQ ID NO: 21, respectively, and HEK293 cells were then transfected with the resulting plasmids to obtain an anti-DLL3 chimeric antibody M6CHI.
Human IgG1 heavy chain constant region (SEQ ID NO: 20):
Human κ light chain constant region (hκ) (SEQ ID NO: 21):



.

### Example 3. Humanization of Anti-DLL3 Monoclonal Antibodies

By aligning the Kabat database of human antibody heavy and light chain variable region germline genes, heavy and light chain variable region germline genes with high homology were selected as templates, respectively, and the CDRs of the murine antibody were grafted onto the corresponding human templates, respectively, to form the variable region sequences in the order of FR1-CDR1-FR2-CDR2-FR3-CDR3-FR4. Certain amino acids in the variable regions were subjected to amino acid substitution, and the resulting variable regions were then recombined with the constant regions (for example, the human IgG1 heavy chain constant region as set forth in SEQ ID NO: 20 and the human κ light chain constant region as set forth in SEQ ID NO: 21) to obtain full-length humanized antibodies.

For the mAb6 antibody, the human germline light chain templates were IGKV1-16*01 or IGKV3-20*02 and IGKJ4*01, and the human germline heavy chain templates were IGHV1-3*01, IGHV7-4-1*02 or IGHV3-73*01 and IGHJ6*01. Optionally, the amino acid residues at positions 1, 36, 42, 43, 44, 46, 52, 56, 69, 71, 79, 85, and/or 94 in the light chain variable region of the humanized antibody were substituted; and/or the amino acid residues at positions 1, 27, 30, 38, 43, 48, 67, 68, 69, 71, 73, 75, 76, 93, 56, 58, and/or 5 in the heavy chain variable region of the humanized antibody were substituted.

**Table 5. Humanization templates and corresponding point mutations for the mAb6 murine antibody**

| hu **VL** | | hu **VH** | |
|---|---|---|---|
| VL1 | Graft(IGKV1-16*01) + F36L, S46G | VH1 | Graft(IGHV1-3*01) + Q1E, R71V, T73K |
| VL2 | Graft(IGKV1-16*01) + F36L, S46G, T69A, F71Y | VH2 | Graft(IGHV1-3*01) + Q1E, I69L, R71V, T73K, S76N |
| VL3 | Graft(IGKV1-16*01) + F36L, A43S, P44F, S46G, T69A, F71Y | VH3 | Graft(IGHV1-3*01) + Q1E, M48I, V67A, I69L, R71V, T73K, S76N |
| VL4 | Graft(IGKV1-16*01) + F36L, S46G, T69A, F71Y, T85D | VH4 | Graft(IGHV1-3*01) + Q1E, Q43K, I69L, R71V, T73K, S76N |
| VL5 | Graft(IGKV1-16*01) | VH5 | Graft(IGHV7-4-1*02) + Q1E, L71V, T73K, F69L |
| VL6 | Graft(IGKV1-16*01) + F36L, S46G, T69A, F71Y, T85D +D56E | VH6 | Graft(IGHV7-4-1*02) + Q1E, F69L, L71V, T73K, V75S, S76N |
| VL7 | Graft(IGKV1-16*01) + F36L, S46G, T69A, F71Y, T85D +D56S | VH7 | Graft(IGHV3-73*01) + F27Y, S30T, I69L, R71V, D73K,T93A |
| VL8 | Graft(IGKV1-16*01) + F36L, S46G, T69A, F71Y, T85D +F94Y, D56E | VH8 | Graft(IGHV1-3*01) |
| VL9 | Graft(IGKV1-16*01) + F36L, S46G, T69A, F71Y, T85D +F94Y, D1N, A52R, D56E | VH9 | Graft(IGHV7-4-1*02) |
| VL10 | Graft(IGKV3-20*02)+ Y36L,Q42K,L46G,T69A,F 71Y,E79Q,V85D + D56E | VH10 | Graft(IGHV3-73*01) |
| VL11 | Graft(IGKV3-20*02) + D56E | VH11 | Graft(IGHV7-4-1*02) + Q1E, R38K, F69L, L71V, T73K,V75S, S76N |
| | | VH12 | Graft(IGHV7-4-1*02) + Q1E, V68A, F69L, L71V, T73K, V75S, S76N |
| | | VH13 | Graft(IGHV7-4-1*02) + Q1E, F69L, L71V, T73K, V75S, S76N, R38K,V68A |
| | | VH14 | Graft(IGHV7-4-1*02) + Q1E, F69L, L71V, T73K, V75S, S76N, -V68A +I58T |
| | | VH15 | Graft(IGHV7-4-1*02) + Q1E, F69L, L71V, T73K, V75S, S76N, -V68A +V56S, V5Q |
| FR4 | IGKJ4*01 | FR4 | IGHJ6*01 |

| | | | |
|---|---|---|---|
| Notes: The amino acid positions in the table are numbered according to the Kabat numbering system. F36L indicates that the F at position 36, according to the Kabat numbering system, is mutated to L. The same applies hereinafter. | | | |

The sequences of the hu6 humanized antibody variable regions obtained are as follows:
hu6 VH1 (SEQ ID NO: 22) 
EVQLVQSGAEVKKPGASVKVSCKASGYTFTDYYMNWVRQAPGQRL
EWMGIINPYSGVTIYNHKFKGRVTITVDKSASTAYMELSSLRSEDTAVYYC
ARQDSLLDYAMDYWGQGTTVTVSS
hu6 VH2 (SEQ ID NO: 23) 
EVQLVQSGAEVKKPGASVKVSCKASGYTFTDYYMNWVRQAPGQRL
EWMGIINPYSGVTIYNHKFKGRVTLTVDKSANTAYMELSSLRSEDTAVYY
CARQDSLLDYAMDYWGQGTTVTVSS
hu6 VH3 (SEQ ID NO: 24) 
EVQLVQSGAEVKKPGASVKVSCKASGYTFTDYYMNWVRQAPGQRL
EWIGIINPYSGVTIYNHKFKGRATLTVDKSANTAYMELSSLRSEDTAVYYC
ARQDSLLDYAMDYWGQGTTVTVSS
hu6 VH4 (SEQ ID NO: 25) 
EVQLVQSGAEVKKPGASVKVSCKASGYTFTDYYMNWVRQAPGKRL
EWMGIINPYSGVTIYNHKFKGRVTLTVDKSANTAYMELSSLRSEDTAVYY
CARQDSLLDYAMDYWGQGTTVTVSS
hu6 VH5 (SEQ ID NO: 26) 
EVQLVQSGSELKKPGASVKVSCKASGYTFTDYYMNWVRQAPGQGLE
WMGIINPYSGVTIYNHKFKGRFVLSVDKSVSTAYLQISSLKAEDTAVYYCA
RQDSLLDYAMDYWGQGTTVTVSS
hu6 VH6 (SEQ ID NO: 27) 
EVQLVQSGSELKKPGASVKVSCKASGYTFTDYYMNWVRQAPGQGLE
WMGIINPYSGVTIYNHKFKGRFVLSVDKSSNTAYLQISSLKAEDTAVYYCA
RQDSLLDYAMDYWGQGTTVTVSS
hu6 VH7 (SEQ ID NO: 28) 
EVQLVESGGGLVQPGGSLKLSCAASGYTFTDYYMNWVRQASGKGLE
WVGIINPYSGVTIYNHKFKGRFTLSVDKSKNTAYLQMNSLKTEDTAVYYC
ARQDSLLDYAMDYWGQGTTVTVSS
hu6VH8 (SEQ ID NO: 29) (Graft IGHV1-3*01) 
QVQLVQSGAEVKKPGASVKVSCKASGYTFTDYYMNWVRQAPGQRL
EWMGIINPYSGVTIYNHKFKGRVTITRDTSASTAYMELSSLRSEDTAVYYC
ARQDSLLDYAMDYWGQGTTVTVSS
hu6VH9 (SEQ ID NO: 30) (Graft IGHV7-4-1*02) 
QVQLVQSGSELKKPGASVKVSCKASGYTFTDYYMNWVRQAPGQGL
EWMGIINPYSGVTIYNHKFKGRFVFSLDTSVSTAYLQISSLKAEDTAVYYC
ARQDSLLDYAMDYWGQGTTVTVSS
hu6VH10 (SEQ ID NO: 31) (Graft IGHV3-73*01) 
EVQLVESGGGLVQPGGSLKLSCAASGFTFSDYYMNWVRQASGKGLE
WVGIINPYSGVTIYNHKFKGRFTISRDDSKNTAYLQMNSLKTEDTAVYYCT
RQDSLLDYAMDYWGQGTTVTVSS
hu6VH11 (SEQ ID NO: 32) 
EVQLVQSGSELKKPGASVKVSCKASGYTFTDYYMNWVKQAPGQGL
EWMGIINPYSGVTIYNHKFKGRFVLSVDKSSNTAYLQISSLKAEDTAVYYC
ARQDSLLDYAMDYWGQGTTVTVSS
hu6VH12 (SEQ ID NO: 33) 
EVQLVQSGSELKKPGASVKVSCKASGYTFTDYYMNWVRQAPGQGLE
WMGIINPYSGVTIYNHKFKGRFALSVDKSSNTAYLQISSLKAEDTAVYYCA
RQDSLLDYAMDYWGQGTTVTVSS
hu6VH13 (SEQ ID NO: 34) 
EVQLVQSGSELKKPGASVKVSCKASGYTFTDYYMNWVKQAPGQGL
EWMGIINPYSGVTIYNHKFKGRFALSVDKSSNTAYLQISSLKAEDTAVYYC
ARQDSLLDYAMDYWGQGTTVTVSS
hu6VH14 (SEQ ID NO: 35) 
EVQLVQSGSELKKPGASVKVSCKASGYTFTDYYMNWVRQAPGQGLE
WMGIINPYSGVTTYNHKFKGRFALSVDKSSNTAYLQISSLKAEDTAVYYC
ARQDSLLDYAMDYWGQGTTVTVSS
hu6VH15 (SEQ ID NO: 36) 
EVQLQQSGSELKKPGASVKVSCKASGYTFTDYYMNWVRQAPGQGLE
WMGIINPYSGSTIYNHKFKGRFALSVDKSSNTAYLQISSLKAEDTAVYYCA
RQDSLLDYAMDYWGQGTTVTVSS
hu6 VL1 (SEQ ID NO: 37) 
DIQMTQSPSSLSASVGDRVTITCHASQGISGNMGWLQQKPGKAPKGLI
YHGANLEDGVPSRFSGSGSGTDFTLTISSLQPEDFATYYCVQYAQFPYTFG
GGTKVEIK
hu6 VL2 (SEQ ID NO: 38) 
DIQMTQSPSSLSASVGDRVTITCHASQGISGNMGWLQQKPGKAPKGLI
YHGANLEDGVPSRFSGSGSGADYTLTISSLQPEDFATYYCVQYAQFPYTFG
GGTKVEIK
hu6 VL3 (SEQ ID NO: 39) 
DIQMTQSPSSLSASVGDRVTITCHASQGISGNMGWLQQKPGKSFKGLI
YHGANLEDGVPSRFSGSGSGADYTLTISSLQPEDFATYYCVQYAQFPYTFG
GGTKVEIK
hu6 VL4 (SEQ ID NO: 40) 
DIQMTQSPSSLSASVGDRVTITCHASQGISGNMGWLQQKPGKAPKGLI
YHGANLEDGVPSRFSGSGSGADYTLTISSLQPEDFADYYCVQYAQFPYTFG
GGTKVEIK
hu6VL5 (SEQ ID NO: 41) (Graft IGKV1-16*01) 
DIQMTQSPSSLSASVGDRVTITCHASQGISGNMGWFQQKPGKAPKSLI
YHGANLEDGVPSRFSGSGSGTDFTLTISSLQPEDFATYYCVQYAQFPYTFG
GGTKVEIK
hu6VL6 (SEQ ID NO: 42) 
DIQMTQSPSSLSASVGDRVTITCHASQGISGNMGWLQQKPGKAPKGLI
YHGANLEEGVPSRFSGSGSGADYTLTISSLQPEDFADYYCVQYAQFPYTFG
GGTKVEIK
hu6VL7 (SEQ ID NO: 43) 
DIQMTQSPSSLSASVGDRVTITCHASQGISGNMGWLQQKPGKAPKGLI
YHGANLESGVPSRFSGSGSGADYTLTISSLQPEDFADYYCVQYAQFPYTFG
GGTKVEIK
hu6VL8 (SEQ ID NO: 44) 
DIQMTQSPSSLSASVGDRVTITCHASQGISGNMGWLQQKPGKAPKGLI
YHGANLEEGVPSRFSGSGSGADYTLTISSLQPEDFADYYCVQYAQYPYTFG
GGTKVEIK
hu6VL9 (SEQ ID NO: 45) 
NIQMTQSPSSLSASVGDRVTITCHASQGISGNMGWLQQKPGKAPKGLI
YHGRNLEEGVPSRFSGSGSGADYTLTISSLQPEDFADYYCVQYAQYPYTFG
GGTKVEIK
hu6VL10 (SEQ ID NO: 46) Graft(IGKV3-20*02)+ Y36L,Q42K,L46G,T69A,F71Y,E79Q,V85D + D56E 
EIVLTQSPATLSLSPGERATLSCHASQGISGNMGWLQQKPGKAPRGLI
YHGANLEEGIPARFSGSGSGADYTLTISRLQPEDFADYYCVQYAQFPYTFG
GGTKVEIK
hu6VL11 (SEQ ID NO: 47) Graft(IGKV3-20*02)+ D56E

EIVLTQSPATLSLSPGERATLSCHASQGISGNMGWYQQKPGQAPRLLI
YHGANLEEGIPARFSGSGSGTDFTLTISRLEPEDFAVYYCVQYAQFPYTFG
GGTKVEIK

Notes: The CDRs are single underlined, the amino acid substitution sites are double underlined, and the rest are FR regions. The same applies hereinafter.

The CDRs of the humanized antibodies of mAb6 are as follows:

**Table 6. CDRs of the humanized antibodies of mAb6**

| Variable region | CDR | Sequence | SEQ ID NO |
|---|---|---|---|
| hu6VH14 | HCDR2 | INPYSGVT**T**YNHKFKG | 48 |
| hu6VH15 | HCDR2 | INPYSG**S**TIYNHKFKG | 49 |
| hu6VL6/VL8/VL10/VL11 | LCDR2 | HGANLE**E** | 50 |
| hu6VL7 | LCDR2 | HGANLE**S** | 51 |
| hu6VL9 | LCDR2 | HG**R**NLE**E** | 52 |
| hu6VL8/VL9 | LCDR3 | VQYAQ**Y**PYT | 53 |

**Table 7. Humanized antibodies of mAb6**

| Variable region | VL1 | VL2 | VL3 | VL4 | VL5 |
|---|---|---|---|---|---|
| VH1 | hu6L1H1 | hu6L2H1 | hu6L3H1 | hu6L4H1 | hu6L5H1 |
| VH2 | hu6L1H2 | hu6L2H2 | hu6L3H2 | hu6L4H2 | hu6L5H2 |
| VH3 | hu6L1H3 | hu6L2H3 | hu6L3H3 | hu6L4H3 | hu6L5H3 |
| VH4 | hu6L1H4 | hu6L2H4 | hu6L3H4 | hu6L4H4 | hu6L5H4 |
| VH5 | hu6L1H5 | hu6L2H5 | hu6L3H5 | hu6L4H5 | hu6L5H5 |
| VH6 | hu6L1H6 | hu6L2H6 | hu6L3H6 | hu6L4H6 | hu6L5H6 |
| VH7 | hu6L1H7 | hu6L2H7 | hu6L3H7 | hu6L4H7 | hu6L5H7 |
| VH8 | hu6L1H8 | hu6L2H8 | hu6L3H8 | hu6L4H8 | hu6L5H8 |
| VH9 | hu6L1H9 | hu6L2H9 | hu6L3H9 | hu6L4H9 | hu6L5H9 |
| VH10 | hu6L1H10 | hu6L2H10 | hu6L3H10 | hu6L4H10 | hu6L5H10 |
| VH11 | hu6L1H11 | hu6L2H 11 | hu6L3H11 | hu6L4H11 | hu6L5H 11 |
| VH12 | hu6L1H12 | hu6L2H 12 | hu6L3H12 | hu6L4H12 | hu6L5H12 |
| VH13 | hu6L1H13 | hu6L2H 13 | hu6L3H13 | hu6L4VH13 | hu6L5H13 |
| VH14 | hu6L1H14 | hu6L2H 14 | hu6L3H14 | hu6L4VH14 | hu6L5H14 |
| VH15 | hu6L1H15 | hu6L2H15 | hu6L3H15 | hu6L4VH15 | hu6L5H15 |

| | | | | | |
|---|---|---|---|---|---|
| Notes: hu6L1H1 indicates that the antibody comprises the heavy chain variable region hu6VH1 and the light chain variable region hu6VL1, and that the sequence of the heavy chain constant region is SEQ ID NO: 20 and the sequence of the light chain constant region is SEQ ID NO: 21. The same applies to the others. | | | | | |

**Table 8. Humanized antibodies of mAb6**

| Variable region | VL6 | VL7 | VL8 | VL9 | VL10 | VL11 |
|---|---|---|---|---|---|---|
| VH1 | hu6L6H1 | hu6L7H1 | hu6L8H1 | hu6L9H1 | hu6L10H1 | hu6L11H1 |
| VH2 | hu6L6H2 | hu6L7H2 | hu6L8H2 | hu6L9H2 | hu6L10H2 | hu6L11H2 |
| VH3 | hu6L6H3 | hu6L7H3 | hu6L8H3 | hu6L9H3 | hu6L10H3 | hu6L11H3 |
| VH4 | hu6L6H4 | hu6L7H4 | hu6L8H4 | hu6L9H4 | hu6L10H4 | hu6L11H4 |
| VH5 | hu6L6H5 | hu6L7H5 | hu6L8H5 | hu6L9H5 | hu6L10H5 | hu6L11H5 |
| VH6 | hu6L6H6 | hu6L7H6 | hu6L8H6 | hu6L9H6 | hu6L10H6 | hu6L11H6 |
| VH7 | hu6L6H7 | hu6L7H7 | hu6L8H7 | hu6L9H7 | hu6L10H7 | hu6L11H7 |
| VH8 | hu6L6H8 | hu6L7H8 | hu6L8H8 | hu6L9H8 | hu6L10H8 | hu6L11H8 |
| VH9 | hu6L6H9 | hu6L7H9 | hu6L8H9 | hu6L9H9 | hu6L10H9 | hu6L11H9 |
| VH10 | hu6L6H10 | hu6L7H10 | hu6L8H10 | hu6L9H10 | hu6L10H10 | hu6L11H10 |
| VH11 | hu6L6H11 | hu6L7H11 | hu6L8H11 | hu6L9H11 | hu6L10H11 | hu6L11H11 |
| VH12 | hu6L6H12 | hu6L7H12 | hu6L8H12 | hu6L9H12 | hu6L10H12 | hu6L11H12 |
| VH13 | hu6L6H13 | hu6L7H13 | hu6L8H13 | hu6L9VH13 | hu6L10H13 | hu6L11H13 |
| VH14 | hu6L6H14 | hu6L7H14 | hu6L8H14 | hu6L9VH14 | hu6L10H14 | hu6L11H14 |
| VH15 | hu6L6H15 | hu6LH15 | hu6L8H15 | hu6L9VH15 | hu6L10H15 | hu6L11H15 |

The above antibodies were cloned, expressed, and purified, respectively, and through a protein binding assay (Test Example 3), a cell binding assay (Test Example 2), and Biacore (Test Example 1), humanized antibodies with good activity were finally selected. The heavy and light chain amino acid sequences of an exemplary humanized antibody are as follows:
hu6 (also known as hu6L4H12) heavy chain (SEQ ID NO: 54): 
EVQLVQSGSELKKPGASVKVSCKASGYTFTDYYMNWVRQAPGQGLE
WMGIINPYSGVTIYNHKFKGRFALSVDKSSNTAYLQISSLKAEDTAVYYCA
RQDSLLDYAMDYWGQGTTVTVSSASTKGPSVFPLAPSSKSTSGGTAALGCLV
KDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKS
RWQQGNVFSCSVMHEALHNHYTQKSLSLSPGK
hu6 (also known as hu6L4H12) light chain (SEQ ID NO: 55):

DIQMTQSPSSLSASVGDRVTITCHASQGISGNMGWLQQKPGKAPKGLI
YHGANLEDGVPSRFSGSGSGADYTLTISSLQPEDFADYYCVQYAQFPYTFG
GGTKVEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKS
FNRGEC

Notes: In the sequences, the variable regions are underlined, and the constant regions are italicized.

### Example 4. Preparation of Anti-DLL3-CD3 Bispecific Antibodies

### 4.1. CD3 Antibodies Used in the Present Invention

The CD3-binding moiety of the present invention may be derived from any suitable antibody. Specifically, S107E was used in the examples of the present invention, and its variable regions and CDR sequences are as follows:

**Table 9. CDRs of S107E**

| Antibody | Name | Sequence | No. |
|---|---|---|---|
| | HCDR1 | KYAMN | SEQ ID NO: 56 |
| | HCDR2 | RIRSKYNNYATYYADSVKD | SEQ ID NO: 57 |
| S107E | HCDR3 | HGNFGN**E**YISYWAY | SEQ ID NO: 58 |
| | LCDR1 | GSSTGAVTSGNYPN | SEQ ID NO: 59 |
| | LCDR2 | GTKFLAP | SEQ ID NO: 60 |
| | LCDR3 | VLWYSNRWV | SEQ ID NO: 61 |

The variable region sequences of S107E are as follows:
> S107E-VH (SEQ ID NO: 62) 
EVQLVESGGGLVQPGGSLKLSCAASGFTFNKYAMNWVRQAPGKGLE
WVARIRSKYNNYATYYADSVKDRFTISRDDSKNTAYLQMNNLKTEDTAV
YYCVRHGNFGNEYISYWAYWGQGTLVTVSS
> S107E-VL (SEQ ID NO: 63)

QTVVTQEPSLTVSPGGTVTLTCGSSTGAVTSGNYPNWVQQKPGQAPR
GLIGGTKFLAPGTPARFSGSLLGGKAALTLSGVQPEDEAEYYCVLWYSNR
WVFGGGTKLTVL

Notes: The CDRs are underlined.

### 4.2. Structure of DLL3-CD3 Bispecific Antibodies of the Present Invention

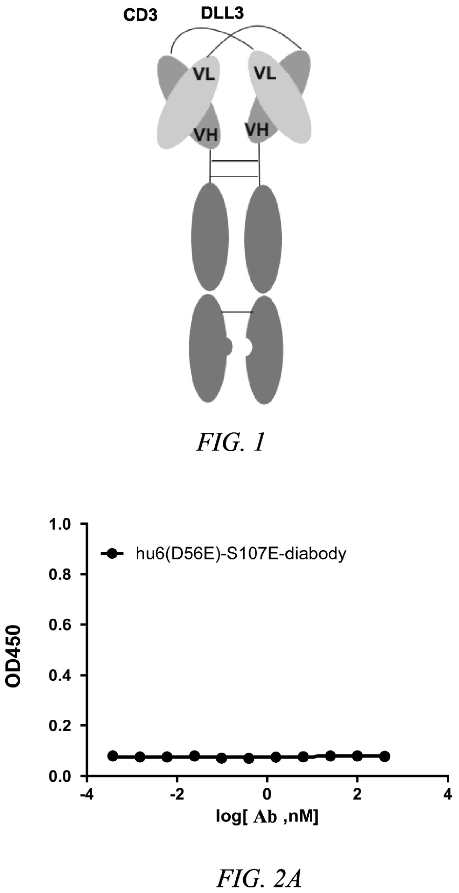

The DLL3-CD3 bispecific antibody molecules of the present invention comprise two chains, the specific structures of which are as follows:
Chain 1 structure: [VL(anti-DLL3)]-Linker 1-[VH(anti-CD3)]-Linker 2-[Fc (Knob, L234A, L235A, G237A, Y349C, T366W)];
Chain 2 structure: [VL(anti-CD3)]-Linker 3-[VH(anti-DLL3)]-Linker 2-[Fc (hole, L234A, L235A, G237A, S354C, T366S, L368A, Y407V)]; and
the schematic diagram of the bispecific antibody molecules is as shown in Figure 1.

The Fc regions of both chains of the bispecific antibody molecules are those capable of associating with each other via the Knob-into-Hole technique. For example, the Fc region of Chain 1 has a knob in accordance with the Knob-into-Hole technique (e.g., SEQ ID NO: 64 or SEQ ID NO: 66), and the Fc region of Chain 2 has a hole in accordance with the Knob-into-Hole technique (e.g., SEQ ID NO: 65 or SEQ ID NO: 67).

The relevant sequences are as follows:
> IgG1Fc(Knob, L234A/L235A/S354C/T366W) (SEQ ID NO: 64) 
DKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNG
KEYKCKVSNKALPAPIEKTISKAKGQPREPQVYTLPPCREEMTKNQVSLW
CLVKGFYPSDIAVEWESNGQPENNYKTTPPVLDSDGSFFLYSKLTVDKSR
WQQGNVFSCSVMHEALHNHYTQKSLSLSPGK
> IgG1Fc(Hole, L234A, L235A, Y349C,T366S, L368A,Y407V) (SEQ ID NO: 65) 
DKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNG
KEYKCKVSNKALPAPIEKTISKAKGQPREPQVCTLPPSREEMTKNQVSLSC
AVKGFYPSDIAVEWESNGQPENNYKTTPPVLDSDGSFFLVSKLTVDKSRW
QQGNVFSCSVMHEALHNHYTQKSLSLSPGK
> Fc Knob(L234A, L235A, G237A, Y349C, T366W) (SEQ ID NO: 66) 
CPPCPAPEAAGAPSVFLFPPKPKDTLMISRTPEVTCVVVDVSHEDPEV
KFNWYVDGVEVHNAKTKPREEQYNSTYRVVSVLTVLHQDWLNGKEYKC
KVSNKALPAPIEKTISKAKGQPREPQVCTLPPSREEMTKNQVSLWCLVKGF
YPSDIAVEWESNGQPENNYKTTPPVLDSDGSFFLYSKLTVDKSRWQQGNV
FSCSVMHEALHNHYTQKSLSLSPGK
> Fc hole(L234A, L235A, G237A, S354C, T366S, L368A, Y407V) (SEQ ID NO: 67) 
CPPCPAPEAAGAPSVFLFPPKPKDTLMISRTPEVTCVVVDVSHEDPEV
KFNWYVDGVEVHNAKTKPREEQYNSTYRVVSVLTVLHQDWLNGKEYKC
KVSNKALPAPIEKTISKAKGQPREPQVYTLPPCREEMTKNQVSLSCAVKGF
YPSDIAVEWESNGQPENNYKTTPPVLDSDGSFFLVSKLTVDKSRWQQGNV
FSCSVMHEALHNHYTQKSLSLSPGK
> Linker 1 (SEQ ID NO: 68)
   GGGSGGGG
> Linker 2 (SEQ ID NO: 69)
   G
> Linker 3 (SEQ ID NO: 70)
   GGGGSGGGG.

Based on the amino acid sequences of the humanized antibodies of mAb6, a bispecific antibody as shown below was constructed:
hu6(D56E)-S107E-diabody
Chain 1: hu6-VL (D56E)-Linker 1-S107E-Linker 2-VH-Fc knob (SEQ ID NO: 71);
Chain 2: CD3VL-Linker 3-hu6VH-Linker 2-Fc hole (SEQ ID NO: 72).

Notes: hu6(D56E)-S107E-diabody indicates that the molecule uses the variable region of the hu6(D56E) humanized antibody as the DLL3-binding domain, the variable region of S107E as the CD3-binding domain, and the diabody as the molecular structure.
> Chain 1 (SEQ ID NO: 71) 
DIQMTQSPSSLSASVGDRVTITCHASQGISGNMGWLQQKPGKAPKGLI
YHGANLEEGVPSRFSGSGSGADYTLTISSLQPEDFADYYCVQYAQFPYTFG
GGTKVEIKGGGSGGGGEVQLVESGGGLVQPGGSLKLSCAASGFTFNKYA
MNWVRQAPGKGLEWVARIRSKYNNYATYYADSVKDRFTISRDDSKNTAY
LQMNNLKTEDTAVYYCVRHGNFGNEYISYWAYWGQGTLVTVSSGCPPCP
APEAAGAPSVFLFPPKPKDTLMISRTPEVTCVVVDVSHEDPEVKFNWYVD
GVEVHNAKTKPREEQYNSTYRVVSVLTVLHQDWLNGKEYKCKVSNKALP
APIEKTISKAKGQPREPQVCTLPPSREEMTKNQVSLWCLVKGFYPSDIAVE
WESNGQPENNYKTTPPVLDSDGSFFLYSKLTVDKSRWQQGNVFSCSVMH
EALHNHYTQKSLSLSPGK
> Chain 2 (SEQ ID NO: 72)

QTVVTQEPSLTVSPGGTVTLTCGSSTGAVTSGNYPNWVQQKPGQAPR
GLIGGTKFLAPGTPARFSGSLLGGKAALTLSGVQPEDEAEYYCVLWYSNR
WVFGGGTKLTVLGGGGSGGGGEVQLVQSGSELKKPGASVKVSCKASGYT
FTDYYMNWVRQAPGQGLEWMGIINPYSGVTIYNHKFKGRFALSVDKSSN
TAYLQISSLKAEDTAVYYCARQDSLLDYAMDYWGQGTTVTVSSGCPPCP
APEAAGAPSVFLFPPKPKDTLMISRTPEVTCVVVDVSHEDPEVKFNWYVD
GVEVHNAKTKPREEQYNSTYRVVSVLTVLHQDWLNGKEYKCKVSNKALP
APIEKTISKAKGQPREPQVYTLPPCREEMTKNQVSLSCAVKGFYPSDIAVE
WESNGQPENNYKTTPPVLDSDGSFFLVSKLTVDKSRWQQGNVFSCSVMH
EALHNHYTQKSLSLSPGK

The control molecule AMG-75 used in the present invention was prepared with reference to WO 2017021349. The sequence of the control molecule is as follows:

AMG-757 (SEQ ID NO: 73):

QVQLQESGPGLVKPSETLSLTCTVSGGSISSYYWSWIRQPPGKCLEWI
GYVYYSGTTNYNPSLKSRVTISVDTSKNQFSLKLSSVTAADTAVYYCASIA
VTGFYFDYWGQGTLVTVSSGGGGSGGGGSGGGGSEIVLTQSPGTLSLSPG
ERVTLSCRASQRVNNNYLAWYQQRPGQAPRLLIYGASSRATGIPDRFSGSG
SGTDFTLTISRLEPEDFAVYYCQQYDRSPLTFGCGTKLEIKSGGGGSEVQLV
ESGGGLVQPGGSLKLSCAASGFTFNKYAMNWVRQAPGKGLEWVARIRSK
YNNYATYYADSVKDRFTISRDDSKNTAYLQMNNLKTEDTAVYYCVRHGN
FGNSYISYWAYWGQGTLVTVSSGGGGSGGGGSGGGGSQTVVTQEPSLTV
SPGGTVTLTCGSSTGAVTSGNYPNWVQQKPGQAPRGLIGGTKFLAPGTPA
RFSGSLLGGKAALTLSGVQPEDEAEYYCVLWYSNRWVFGGGTKLTVLGG
GGDKTHTCPPCPAPELLGGPSVFLFPPKPKDTLMISRTPEVTCVVVDVSHE
DPEVKFNWYVDGVEVHNAKTKPCEEQYGSTYRCVSVLTVLHQDWLNGK
EYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQ
QGNVFSCSVMHEALHNHYTQKSLSLSPGGGGSGGGGSGGGGSGGGGSGG
GGSGGGGSDKTHTCPPCPAPELLGGPSVFLFPPKPKDTLMISRTPEVTCVVV
DVSHEDPEVKFNWYVDGVEVHNAKTKPCEEQYGSTYRCVSVLTVLHQD
WLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVD
KSRWQQGNVFSCSVMHEALHNHYTQKSLSLSPGK

### Test Examples

### Test Example 1: Biacore Antibody Affinity Assay

Test antibodies were affinity-captured with a Protein A biosensor chip (Cat. # 29127556, Cytiva) for 18 seconds, and then the antigens human DLL3 (ACRO, DLL3-H52H4), cynomolgus DLL3 (KACTUS, DLL-RM103), mouse DLL3 (KACTUS, DLL-MM103), human CD3D&3E (ACRO, CDD-H52W1), and cynomolgus CD3D&3E (ACRO, CDD-C52W4) were allowed to flow through the surface of the chip for 180 seconds, followed by 600 seconds of dissociation. Reaction signals were detected in real time using a Biacore 8K (Cytiva) instrument to obtain association and dissociation curves. After the dissociation in each experimental cycle, the chip was washed clean and regenerated with a 10 mM glycine-hydrochloric acid solution (pH 1.5, Cat. # BR-1003-54, Cytiva). Data fitting was performed using a 1 : 1 Model. The results are as shown in Table 10 and Table 11.

**Table 10. Affinities of the antibodies for human DLL3 (based on Clone 6)**

| **Antibody** | **ka (1/Ms)** | **kd (1/s)** | **KD (M)** |
|---|---|---|---|
| M6CHI | 9.65E+05 | 6.57E-04 | 6.81E-10 |
| hu6L1H1 | 7.98E+05 | 2.25E-03 | 2.81E-09 |
| hu6L1H2 | 7.87E+05 | 1.73E-03 | 2.19E-09 |
| hu6L1H3 | 8.12E+05 | 2.24E-03 | 2.76E-09 |
| hu6L2H1 | 7.09E+05 | 1.55E-03 | 2.19E-09 |
| hu6L2H2 | 7.04E+05 | 1.16E-03 | 1.64E-09 |
| hu6L2H3 | 6.95E+05 | 1.39E-03 | 2.01E-09 |
| hu6L3H1 | 7.15E+05 | 1.44E-03 | 2.02E-09 |
| hu6L3H2 | 7.16E+05 | 1.14E-03 | 1.59E-09 |
| hu6L3H3 | 7.18E+05 | 1.35E-03 | 1.88E-09 |
| hu6L4H4 | 7.43E+05 | 1.08E-03 | 1.45E-09 |
| hu6L2H5 | 8.10E+05 | 7.84E-04 | 9.69E-10 |
| hu6L2H6 | 8.63E+05 | 7.56E-04 | 8.77E-10 |
| hu6L2H7 | 6.70E+05 | 2.05E-03 | 3.06E-09 |
| hu6L2H11 | 2.06E+06 | 7.49E-04 | 3.63E-10 |
| hu6L2H12 | 1.98E+06 | 9.13E-04 | 4.61E-10 |
| hu6L2H13 | 2.10E+06 | 8.03E-04 | 3.82E-10 |
| hu6L4H6 | 1.91E+06 | 8.11E-04 | 4.24E-10 |
| hu6L4H11 | 2.14E+06 | 6.72E-04 | 3.14E-10 |
| hu6L4H12 | 2.06E+06 | 8.05E-04 | 3.91E-10 |
| hu6L4H13 | 2.16E+06 | 6.95E-04 | 3.22E-10 |

**Table 11. Affinities of the bispecific antibody hu6(D56E)-S107E-diabody for antigens of different species**

| Antigens of different species | **ka (1/Ms)** | **kd (1/s)** | **KD (M)** |
|---|---|---|---|
| Human DLL3 | 4.22E+05 | 4.62E-04 | 1.09E-09 |
| Cynomolgus DLL3 | 2.64E+05 | 4.88E-04 | 1.85E-09 |
| Mouse DLL3 | 3.78E+05 | 4.51E-03 | 1.19E-08 |
| Human CD3 | 1.93E+05 | 1.12E-02 | 5.82E-08 |
| Cynomolgus CD3 | 2.66E+05 | 1.23E-02 | 4.60E-08 |

The results show that all the humanized antibodies and chimeric antibodies specifically binding to DLL3 of the present invention exhibit good affinities for human DLL3; and the bispecific antibody exhibits good affinities for both human and cynomolgus DLL3 and CD3.

### Test Example 2: DLL3 Cell-Level FACS Binding Assay

Cells of small cell lung cancer cell lines H1184 (ATCC, Cat. No. CRL-5858), DLL3/H82 and DLL3/SHP77, and hDLL3/CHO-S and cynoDLL3/CHO-S cells expressing DLL3, as well as CD3-expressing Jurkat cells (ATCC, TIB-152) were suspended in an FACS buffer (1% BSA + pH 7.4 PBS) to form 1 × 10⁶/mL cell suspensions, and the suspensions were added to 96-well round-bottom plates at 100 µL/well. The plates were centrifuged at 300 g for 5 minutes, and the supernatants were removed. Test antibodies at different concentrations were added at 100 µL/well. The plates were incubated in the dark in a refrigerator at 4°C for 1 hour. After centrifugation at 300 g and washing three times, APC anti-human IgG Fc (BioLegend, 410712) or PE F(ab')2-goat anti-human IgG (Invitrogen, H10104) at working concentration was added. The plates were incubated in the dark in a refrigerator at 4°C for 40 minutes. After centrifugation at 300 g and washing three times, the geometric mean fluorescence intensity was measured on a flow cytometer (Invitrogen), and the EC₅₀ values for the binding of the antibodies to DLL3-expressing cells were calculated. The results are as shown in Table 12 and Table 13.

**Table 12. Binding activities of the antibodies to H1184 cells expressing human DLL3**

| **Antibody** | **Emax** | **EC₅₀ (nM)** |
|---|---|---|
| hu6L2H2 | 2193 | 0.06 |
| hu6L4H4 | 2312 | 0.06 |
| hu6L2H5 | 2486 | 0.06 |
| hu6L2H6 | 2422 | 0.05 |
| hu6L2H7 | 2196 | 0.07 |
| hu6L2H11 | 1959 | 0.04 |
| hu6L2H12 | 2145 | 0.10 |
| hu6L2H13 | 2105 | 0.10 |
| hu6L4H11 | 2188 | 0.07 |
| hu6L4H12 | 2131 | 0.05 |
| hu6L4H13 | 2118 | 0.09 |

**Table 13. Binding activities of the bispecific antibody to cells**

| **Antibody** | **hDLL3/CHO-S EC₅₀ (nM)** | **cynoDLL3/CHO-S EC₅₀ (nM)** | **Jurkat EC₅₀ (nM)** |
|---|---|---|---|
| hu6(D56E)-S107E-diabody | 1.00 | 1.17 | 13.62 |

The results show that the anti-DLL3 humanized antibodies of the present invention exhibit good capacities to bind to cells expressing human DLL3; and the bispecific antibody exhibits good capacities to bind to cells expressing human and cynomolgus DLL3 and CD3.

### Test Example 3: DLL3 Protein-Level ELISA Binding Assay

Plates were coated with streptavidin (Abcam, ab136200, 1 µg/ml) at 100 µL/well, and incubated overnight at 4°C. The plates were washed three times with 250 µL of PBST solution (0.1% Tween 20 in PBS) per well. The plates were blocked with 250 µL of 5% milk per well at 37°C for 2 hours. The plates were washed three times with 250 µL of PBST solution per well. Biotinylated DLL3 antigen (1 µg/mL) (SEQ ID NO: 9) was added, and the plates were incubated at 37°C for 1 hour. The plates were washed three times with 250 µL of PBST solution per well. The antibody (maximum concentration 400 nM, serially diluted 4-fold) was formulated, and incubation was performed at 37°C for 1 hour. The plates were washed six times with 250 µL of PBST solution per well. Human IgG (H+L)-HRP antibody (Jackson, 109-035-003, 1:4000 dilution) at working concentration was added at 100 µL/well, and the plates were incubated at 37°C for 1 hour. The plates were washed six times with 250 µL of PBST solution per well. TMB substrate solution (KPL, 5120-0077) was added at 100 µL/well, and color development was performed at room temperature for 5-10 minutes. 1 M H₂SO₄ was added at 100 µL/well to stop the color development, and the plates were read on a microplate reader (Molecular Devices, VERSA max) at 450 nm.

**Table 14. Binding activity of the bispecific antibody to human DLL3 protein**

| **Antibody** | **EC₅₀ (nM)** | **Emax** |
|---|---|---|
| hu6(D56E)-S107E-diabody | 0.49 | 0.33 |

The results show that the bispecific antibody of the present invention exhibits good affinity for human DLL3 protein.

### Test Example 4: ELISA Binding Assay with DLL1 and DLL4 Proteins

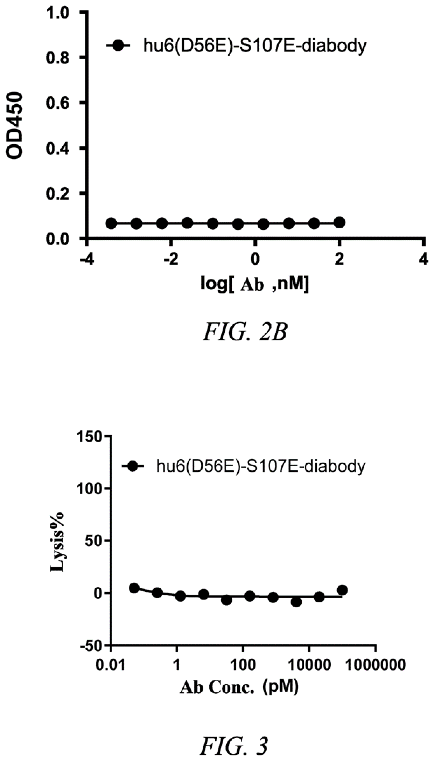

Plates were coated with DLL1 (ACRO, DL1-H52H8, 1 µg/mL) and DLL4 (ACRO, DL4-H5227, 1 µg/mL) at 100 µL/well, and incubated overnight at 4°C. The plates were washed three times with 250 µL of PBST solution (0.1% Tween 20 in PBS) per well. The plates were blocked with 250 µL of 5% milk per well at 37°C for 2 hours. The plates were washed three times with 250 µL of PBST solution per well. The test antibody (maximum concentration 100 nM, serially diluted 4-fold) was added, and the plates were incubated at 37°C for 1 hour. The plates were washed six times with 250 µL of PBST solution per well. Human IgG (H+L)-HRP antibody (Jackson, 109-035-003, 1:4000 dilution) at working concentration was added at 100 µL/well, and the plates were incubated at 37°C for 1 hour. The plates were washed six times with 250 µL of PBST solution per well. TMB substrate solution (KPL, 5120-0077) was added at 100 µL/well, and color development was performed at room temperature for 5-10 minutes. 1 M H₂SO₄ was added at 100 µL/well to stop the color development, and the plates were read on a microplate reader (Molecular Devices, VERSA max) at 450 nm. The results are as shown in Figures 2A-2B.

The results show that the binding of the bispecific antibody of the present invention to human DLL1 and DLL4 proteins is undetectable.

### Test Example 5. Killing Activity of Bispecific Antibody of the Present Invention Against Cells

In this test example, the killing activities of the bispecific antibody as a T cell engager molecule against tumor cells were studied. The target-specific cytotoxic activity of the bispecific antibody of the present invention was detected, with the stably transfected cell lines SHP77, H1184, and H460 expressing LUC-GFP as target cells.

SHP77/lucG cells were cultured in 1640 + 10% FBS + 10 µg/mL puromycin complete culture medium and passaged twice to three times a week. H1184/lucG cells were cultured in 1640 + 5% FBS + 10 µg/mL puromycin complete culture medium and passaged once a week. H460/lucG cells were cultured in 1640 + 10% FBS complete culture medium and passaged twice to three times a week. Cryopreserved PBMCs (MileCell Bio) were thawed and then resuspended in 1640 + 10% FBS complete culture medium. The suspension was cultured in a T75 culture flask for 4 hours (at a density of 2E6 cells/mL). PBMCs were collected, centrifuged, then resuspended in 1640 + 10% FBS, and counted, and the cell count was adjusted to 1.5E6 cells/mL. The PBMC suspension and target cell suspension described above were mixed in equal volumes, and the mixture was added at 100 µL/well to ensure that the E : T Ratio was 10 : 1. In addition, a PBMC ONLY group was set up, in which the above PBMC suspension and 1640 + 10% FBS complete culture medium were mixed in equal volumes. The antibody was 5-fold diluted with 1640 + 10% FBS culture medium with an initial concentration of 600 nM (6× the final concentration), 9 dose points, and 20 µL/well. The treated cells were cultured in a 37°C, 5% CO₂ incubator for 48 hours. The culture plates were taken out and centrifuged at 1000 rpm for 3 minutes. 50 µL of the supernatant was pipetted into a new 3788 plate for cytokine detection, and the remainder was stored at -20°C. 50 µL of ONE-GloTMLuciferase (Promega, E6120) was added to the culture plates. After incubation at room temperature for 5 minutes, luminescence was measured using Vendor, and the killing effects of the antibody at different concentrations were calculated. Dose-response curves were created based on the logarithmic concentrations of the antibody and the signal values using Graphpad Prism8.0 software, and the IC50 values for antibody-mediated killing were obtained. The wells containing only the target cells and PBMCs with no antibody added were set to 0% inhibition, and the maximum inhibition rate Imax was calculated for the antibody. The results are as shown in Table15 and Figure 3.

**Table 15. Cytotoxic activities of the bispecific antibody hu6(D56E)-S107E-diabody against different target cells**

| Target cell | **Imax (%)** | **IC₅₀ (pM)** |
|---|---|---|
| SHP77 | 96 | 83.04 |
| H1184 | 97 | 19.68 |

The results show that the bispecific antibody of the present invention exhibits strong killing effects against the DLL3-expressing cells SHP77 and H1184 and exhibits no killing effects against H460 cells not expressing DLL3 (Figure 3).

### Test Example 6. In Vitro Activation Activities of Bispecific Antibody of the Present Invention on T cells

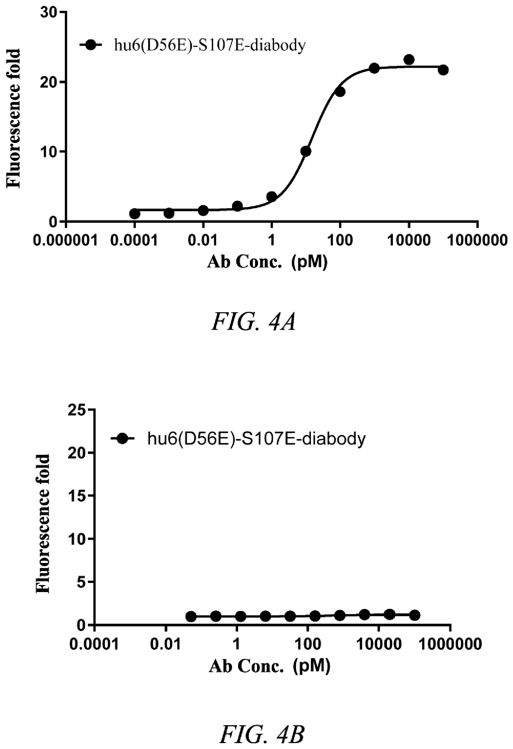

H82/DLL3 cells were cultured in 1640 + 10% FBS complete culture medium and passaged twice to three times a week with a passage ratio of about 1 : 3. H460 cells were cultured in 1640 + 10% FBS complete culture medium and passaged twice to three times a week with a passage ratio of about 1 : 10. Jurkat Lucia^{™} NFAT cells (InvivoGen) were cultured in 1640 + 10% FBS + 100 µg/ml zeocin (InvivoGen, ant-zn-1) complete culture medium and passaged twice to three times a week with a passage ratio of about 1 : 10. Jurkat Lucia^{™} NFAT cells were collected, centrifuged at 1000 rpm for 3 minutes, resuspended, and counted, and the cell count was adjusted to 1E6 cells/mL. Target cells H82/DLL3 cells were collected, centrifuged at 1000 rpm for 3 minutes, resuspended, and counted, and the cell count was adjusted to 2E5 cells/mL. H82/DLL3 and H460 cells were collected, centrifuged at 1000 rpm for 3 minutes, resuspended, and counted, and the cell count was adjusted to 4E5 cells/mL. The Jurkat Lucia^{™} NFAT and target cells described above were mixed in equal volumes, and the mixture was added at 100 µL/well to ensure that the E : T Ratio for H82/DLL3 cells was 5 : 1 and the E : T Ratio for H82/DLL3 and H460 cells was 2.5 : 1. The antibody was 5-fold diluted with 1640 + 10% FBS culture medium with an initial concentration of 600 nM (6× the final concentration), 9 dose points, and 20 µL/well. The treated cells were cultured in a 37°C, 5% CO₂ incubator for 5 hours. QUANTI-Luc^{™} Gold reagent (Invivogen, rep-qlcg5) was dissolved in 25 mL of ddH₂O. 50 µL of QUANTI-Luc^{™} Gold was added. After incubation at room temperature for 5 minutes, luminescence was measured using Vendor, and the activating effects of the antibody at different concentrations on Jurkat Lucia^{™} NFAT cells were calculated. Plots were created using Graphpad Prism8.0 software. The results are as shown in Figures 4A to 4B and Table 16. 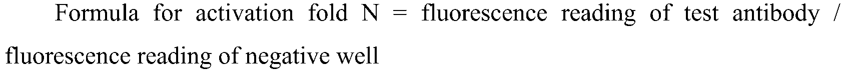 Formula for activation fold N = fluorescence reading of test antibody / fluorescence reading of negative well

**Table 16. Results for the activation of T cells by the bispecific antibody**

| **Antibody** | **Activation fold** | **EC₅₀ (pM)** |
|---|---|---|
| hu6(D56E)-S107E-diabody | 22.20 | 15.21 |

The results show that the bispecific antibody of the present invention activates T cells only in the presence of DLL3-expressing DLL3/H82 cells and does not activate T cells in the presence of H460 negative cells not expressing DLL3.

### Test Example 7. Cytokine Release Levels for Bispecific Antibody of the Present Invention

### 1. Release Levels of Cytokine IFNγ for Bispecific Antibody of the Present Invention

The secretion of cytokine IFNγ in the stimulation of PBMCs with the DLL3/CD3 bispecific antibody in the presence of H1184 cells was detected by HTRF.

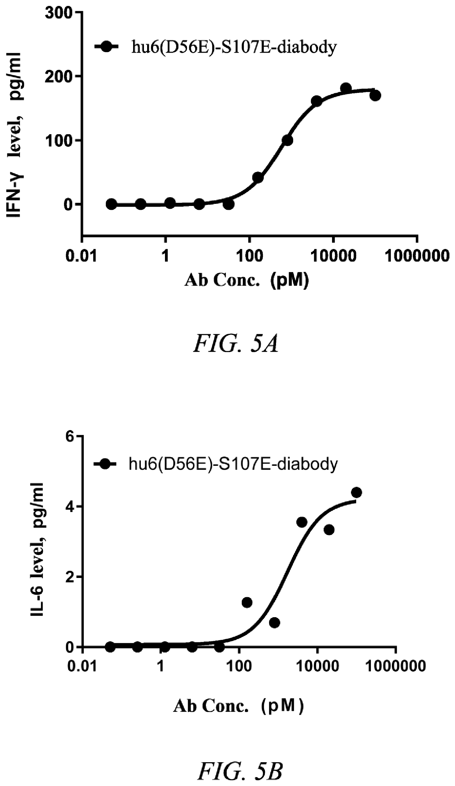

The supernatants collected in the cell killing experiment (Test Example 5), cryopreserved in a freezer at -20°C, were taken out, thawed at room temperature, and shaken for good uniformity. Powdered IFNγ standard was dissolved in sterile water, and the solution of the standard was diluted with 1640 + 10% FBS culture medium to the desired concentration. A kit for IFNγ detection (CISBIO, 62HIFNGPEG) was taken out and equilibrated to room temperature, and two detection antibodies in the kit were 20-fold diluted with a detection buffer. 16 µL of a cell supernatant (10-fold diluted for IFNγ detection) and the IFNγ standard were added to 96-well plates, and 4 µL of the diluted corresponding detection antibody was added. The plates were shaken for good uniformity, centrifuged at 1000 rpm for 1 minute, and incubated overnight at room temperature. The samples incubated overnight were taken out and centrifuged at 1000 rpm for 1 minute, and the absorbance values at 665 nm and 620 nm were read using a PHERAstar multimode microplate reader. Data were analyzed using Graphpad Prism 8. The results are as shown in Figure 5A.

The results show that the bispecific antibody of the present invention causes very low levels of IFNγ release.

### 2. Release Levels of Cytokine IL-6 for Bispecific Antibody of the Present Invention

The secretion of cytokine IL-6 in the stimulation of PBMCs with the DLL3/CD3 bispecific antibody in the presence of H1184 cells was detected by ELISA.

The supernatants collected in the cell killing experiment (Test Example 5), cryopreserved in a freezer at -20°C, were taken out, thawed at room temperature, and shaken for good uniformity. The samples were 5-fold diluted with a sample diluent for later use. Before cytokine detection, an IL-6 ELISA kit (NeoBioscience Technology Co., Ltd., EHC007.96) was taken out and equilibrated to room temperature. IL-6 standard was diluted and detection was performed according to the instructions, respectively. OD450 values were measured, and data were analyzed using Graphpad Prism 8. The results are as shown in Figure 5B.

The results show that the bispecific antibody of the present invention causes very low levels of IL6 release, indicating that the bispecific antibody of the present invention exhibits better safety.

### Evaluation of In Vivo Activity

### Test Example 8. Efficacy of Bispecific Antibody of the Present Invention in SHP77 Subcutaneous Xenograft Tumor Model

In the present invention, severe combined immunodeficiency NOG mice were inoculated with SHP-77 cells, then intraperitoneally injected with human PBMCs, and after tumorigenesis, dosed in groups.

Female NOG mice weighing about 15-17 g were purchased from Vital River. SHP-77 cells were from ATCC. Human PBMCs were purchased from MileCell (Shanghai) Biotechnology Co. Ltd., Cat. No: ID # A10S115034.

100 µL of SHP-77 cells (4 × 10⁶ cells/mouse) were inoculated subcutaneously into the right flank of NOG mice. On the day of inoculation, cryopreserved hPBMCs were thawed. On Day 2 post-inoculation, each mouse was intraperitoneally injected with a cell suspension of hPBMCs (5 × 10⁶ cells/100 µL). Eight days after inoculation, after the tumor volume was ~120 mm³, mice with abnormally high or low body weight or tumor volume were excluded. Mice were randomized into 5 groups of 8 mice according to tumor volume, and administration of an equimolar amount of antibody was started on the day of grouping. The doses are as shown in Table 32. The corresponding equimolar amount of antibody was administered by intraperitoneal injection once every 5 days for a total of three times until Day 15. The tumor volume and body weight were measured twice a week, and data were recorded. $Tumor volume \left(V\right) was calculated using the formula : V = 1 / 2 \times {L}_{long} \times {{L}_{short}}^{2}$ $Relative tumor proliferation rate T / C \left(%\right) = \left(T-{T}_{0}\right) / \left(C-{C}_{0}\right) \times 100 % ,$ where T and C represent the tumor volumes of the treatment group and the control group at the end of the experiment; and T₀ and C₀ represent the tumor volumes at the beginning of the experiment. $Tumor growth inhibition TGI \left(%\right) = 100 - T / C \left(%\right) .$

**Table 17. Efficacy of the antibody in the SHP77 subcutaneous xenograft tumor model (Day 15 post-dose)**

| Group | Dose (mg/kg) | Tumor growth inhibition TGI (%) |
|---|---|---|
| Vehicle | / | / |
| AMG-757 | 1 | 87 |
| hu6(D56E)-S107E-diabody | 0.96 | 107 |

The results show that the bispecific antibody of the present invention can significantly inhibit the growth of SHP-77 xenograft tumors.

### Test Example 9. Efficacy of Bispecific Antibody of the Present Invention in H1184 Subcutaneous Xenograft Tumor Model

In the present invention, severe combined immunodeficiency NOG mice were inoculated with H1184 cells, then intraperitoneally injected with human PBMCs, and after tumorigenesis, dosed in groups.

Female NOG mice weighing about 15-17 g were purchased from Vital River. H1184 cells were from ATCC. Human PBMCs were purchased from MileCell (Shanghai) Biotechnology Co. Ltd., Cat. No: ID # A10S115034.

200 µL of H1184 cells (3.5 × 10⁶ cells, containing 50% MatriGel) were inoculated subcutaneously into the right flank of each NOG mouse. On Day 11 post-inoculation, 8 vials of cryopreserved hPBMCs (ID # A10S115034) were thawed. On Day 12 post-inoculation, each mouse was intraperitoneally injected with a cell suspension of human PBMCs (5 × 10⁶ cells/100 µL). Eighteen days after inoculation, after the tumor volume was ~140 mm³, mice with abnormally high or low body weight or tumor volume were excluded. Mice were randomized into groups of 8 mice according to tumor volume, and administration of an equimolar amount of antibody was started on the day of grouping. The doses are as shown in Table 33. The antibody was administered by intraperitoneal injection twice a week for a total of four times. The tumor volume and body weight were measured twice a week, and data were recorded.

Data were recorded using Excel statistical software: the average values were calculated as avg; the SD values were calculated as STDEV; the SEM values were calculated as STDEV/SQRT (number of animals per group); and GraphPad Prism software was used for plotting, and statistical analysis of the data was performed using Two-way ANOVA, One-way ANOVA, and t-test. $Tumor volume \left(V\right) was calculated using the formula : V = 1 / 2 \times {L}_{long} \times {{L}_{short}}^{2}$ $Relative tumor proliferation rate T / C \left(%\right) = \left(T-{T}_{0}\right) / \left(C-{C}_{0}\right) \times 100 % ,$ where T and C represent the tumor volumes of the treatment group and the control group at the end of the experiment; and T₀ and C₀ represent the tumor volumes at the beginning of the experiment. $Tumor growth inhibition TGI \left(%\right) = 100 - T / C \left(%\right) .$

**Table 18. Efficacy of the antibody in the H1184 subcutaneous xenograft tumor model (Day 15 post-dose)**

| Group | Dose (mg/kg) | Tumor growth inhibition TGI (%) |
|---|---|---|
| Vehicle | / | / |
| AMG-757 | 1 | 13 |
| hu6(D56E)-S107E-diabody | 0.96 | 55 |

The results show that the bispecific antibody of the present invention can significantly inhibit the growth of H1184 xenograft tumors.

### II. Formulation Examples-Bispecific Antibody Formulations Specifically Binding to DLL3 and CD3

### Size Exclusion Chromatography (SEC):

This is an analytical method that separates a solute based on the relative relationship between the pore size of the gel pores and the coil size of the polymer sample molecule. $SEC % \left(SEC monomer content percentage\right) = Amonomer / Atotal \times 100 %$ (Amonomer represents the peak area of the main peak monomer in the sample, and Atotal represents the sum of areas of all peaks). ΔSEC% = SEC% of formulation before stability experiment - SEC% of formulation after stability experiment.

Instruments for SEC analysis: Agilent HPLC 1260; and
Column: Waters, BioResolve^{TM} SEC mAb 200Å, 2.5 µm 7.8 × 300 mm Column.

### Non-reduced Capillary Electrophoresis (NR-CE):

This is a type of electrophoresis in which a gel is transferred into a capillary as a support medium and a method that achieves separation under a certain voltage based on the molecular weight of the sample. $NR - CE % \left(NR-CE main peak content percentage\right) = Amain peak / Atotal \times 100 %$ (Amain peak represents the peak area of the main peak in the sample, and Atotal represents the sum of areas of all peaks). ΔNR-CE% = NR-CE% of formulation before stability experiment - NR-CE% of formulation after stability experiment.

Instrument for CE analysis: Beckman capillary electrophoresis system, model: PA800 plus.

### Imaged Capillary Isoelectric Focusing (iCIEF):

This is a technique for separation according to the difference in isoelectric points (pI) of proteins.

iCIEF% (iCIEF neutral peak content percentage) = Aneutral peak area/Atotal area × 100% (Atotal area represents the sum of areas of acidic, neutral, and basic peaks). ΔiCIEF% = iCIEF% of formulation before stability experiment - iCIEF% of formulation after stability experiment.

Instrument for iCIEF analysis: simple protein, model: Muarice.

### Ion Exchange Chromatography (IEC):

This is a chromatographic method that uses an ion exchange resin or chemically bonded ion exchanger as the stationary phase and achieves separation based on differences in ion exchange ability or selectivity coefficient among the components to be separated. 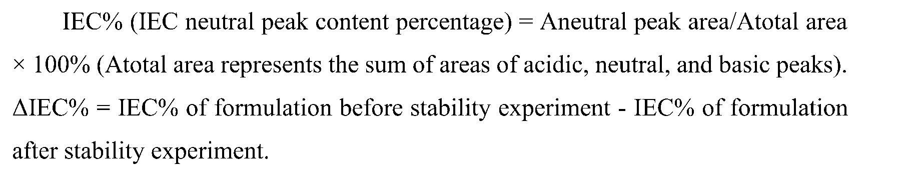 IEC% (IEC neutral peak content percentage) = Aneutral peak area/Atotal area × 100% (Atotal area represents the sum of areas of acidic, neutral, and basic peaks). ΔIEC% = IEC% of formulation before stability experiment - IEC% of formulation after stability experiment.

Instrument for IEC analysis: Agilent HPLC 1260.

### Osmotic pressure determination:

The freezing point method is used for determining osmotic pressure. On the basis of the directly proportional relationship between the freezing point depression value and the molar concentration of a solution, the freezing point of the solution is determined using a highly sensitive temperature-sensing element and then converted into the osmotic pressure via an electrical signal.

Instrument for osmotic pressure determination: Loser, model: OM815.

### Protein

The protein used in the following examples was the bispecific antibody protein specifically binding to DLL3 and CD3 (hu6(D56E)-S107E-diabody, hereinafter referred to as "protein").

Instrument for concentration determination of fusion protein: ultraviolet-visible spectrophotometer, model: Nano Drop oneC, optical path length: 1 mm.

### Formulation Example 1. Screening of pH Values and Buffer Systems

Formulations comprising 70 mg/mL of protein, 80 mg/mL of sucrose, and 0.4 mg/mL of Polysorbate 80 (PS80) were prepared using the buffer systems as shown in Table 19. Forced degradation studies were performed on the samples (storage at 40°C for 4 weeks), with SEC and IEC as evaluation indicators to further investigate the effects of different pH values and buffer systems on protein stability.

The results are as shown in Table 19. The SEC data showed that ΔSEC ranged from 0.8% to 3.2%, with the His-HCl system (pH 4.5-5.5) and His-AA system (pH 5.0) groups being superior. The ΔIEC data showed that the His-HCl system (pH 4.5-5.5) and CA system (pH 5.5) groups were superior. Based on the combined ΔSEC and ΔIEC data, the His-HCl system (pH 4.5-5.5) is preferred.

**Table 19. Results of pH and buffer system screening**

| Group | Buffer system | Condition | ΔSEC% | ΔIEC% |
|---|---|---|---|---|
| 1 | 20 mM CA pH 5.5 | 40°C W4 | 1.9 | 26.3 |
| 2 | 20 mM SA pH 5.5 | 40°C W4 | 2.1 | 28.9 |
| 3 | 20 mM His-HCl pH 4.5 | 40°C W4 | 1.1 | 23.6 |
| 4 | 20 mM His-HCl pH 5.0 | 40°C W4 | 0.8 | 25.6 |
| 5 | 20 mM His-HCl pH 5.5 | 40°C W4 | 1.5 | 25.3 |
| 6 | 20 mM His-HCl pH 6.0 | 40°C W4 | 3.2 | 29.0 |
| 7 | 20 mM His-AA pH 4.5 | 40°C W4 | 2.0 | 32.0 |
| 8 | 20 mM His-AA pH 5.0 | 40°C W4 | 0.9 | 31.9 |

| | | | | |
|---|---|---|---|---|
| Notes: His-HCl: histidine-histidine hydrochloride; SA: succinic acid-sodium succinate; CA: citric acid-sodium citrate; His-AA: histidine-histidine acetate; and 40°C W4: storage at 40°C for 4 weeks. The same applies hereinafter. | | | | |

### Formulation Example 2. Screening of Protein Concentrations

Formulations comprising 0.1 mg/mL of ethylenediaminetetraacetate dihydrate (containing two molecules of water, manufacturer: Hunan Er-Kang Pharmaceutical Co., Ltd., hereinafter referred to as EDTA-2Na, CAS: 6381-92-6), 80 mg/mL of sucrose, 0.4 mg/mL of PS80, and protein at concentrations as shown in Table 20 were prepared with 18 mM His-HCl buffer (pH 5.0). Forced degradation studies were performed on the samples (storage at 40°C for 4 weeks), with SEC and IEC as evaluation indicators to investigate the effects of different protein concentrations on protein stability.

The results are as shown in Table 20. After storage at 40°C for 4 weeks, ΔSEC showed a slight increasing trend with the elevation of protein concentration, while small differences in ΔIEC were observed among the groups.

**Table 20. Results of protein concentration screening**

| Group | Protein concentration (mg/mL) | Storage condition | ΔSEC% | ΔIEC% |
|---|---|---|---|---|
| 1 | 1 | 40°C W4 | -0.5 | 23.9 |
| 2 | 5 | 40°C W4 | 0.2 | 24.1 |
| 3 | 70 | 40°C W4 | 0.8 | 25.6 |
| 4 | 100 | 40°C W4 | 1.5 | 23.7 |
| 5 | 150 | 40°C W4 | 3.6 | 22.0 |

### Formulation Example 3. Screening of EDTA-2Na Concentrations

Formulations comprising 100 mg/mL of protein, 80 mg/mL of sucrose, 0.4 mg/mL of PS80, and EDTA-2Na at different concentrations as shown in Table 21 were prepared with 18 mM His-HCl buffer (pH 5.0). Forced degradation studies were performed on the samples (storage at 40°C for 4 weeks), with SEC, NR-CE, and IEC as evaluation indicators to investigate the effects of different EDTA-2Na concentrations on formulation stability.

The results are as shown in Table 21. After storage at 40°C for 4 weeks, ΔSEC ranged from 0.7% to 1.4%, and the formulation comprising 0.1 mg/mL of EDTA-2Na showed slightly better monomer purity than the other groups. No significant differences in ΔNR-CE and ΔIEC were observed among the groups.

**Table 21. Results of EDTA-2Na concentration screening**

| Group | EDTA-2Na concentration (mg/mL) | Storage condition | ΔSEC% | ΔNR-CE% | ΔIEC% |
|---|---|---|---|---|---|
| 1 | 0.01 | 40°C W4 | 1.3 | 1.9 | 24.5 |
| 2 | 0.1 | 40°C W4 | 0.7 | 1.7 | 24.5 |
| 3 | 1.0 | 40°C W4 | 1.4 | 1.8 | 23.8 |

### Formulation Example 4. Screening of Ionic Strengths of His-HCl Buffer System

Formulations comprising 100 mg/mL of protein, 0.1 mg/mL of EDTA-2Na, 80 mg/mL of sucrose, and 0.4 mg/mL of PS80 were prepared with His-HCl buffer (pH 5.0) at different ionic strengths as shown in Table 22. Forced degradation studies were performed on the samples (storage at 40°C for 4 weeks), with SEC, NR-CE, and IEC as evaluation indicators to investigate the effects of different histidine ionic strengths on protein stability.

The results are as shown in Table 22. After storage at 40°C for 4 weeks, no significant differences in ΔSEC, ΔNR-CE, and ΔIEC were observed among the groups.

**Table 22. Results of His-HCl buffer system concentration screening**

| Group | Ionic strength of His-HCl buffer system | Storage condition | ΔSEC% | ΔNR-CE% | ΔIEC% |
|---|---|---|---|---|---|
| 1 | 10 | 40°C W4 | 1.1 | 1.4 | 23.1 |
| 2 | 18 | 40°C W4 | 0.7 | 1.7 | 24.5 |
| 3 | 50 | 40°C W4 | 1.1 | 1.5 | 23.2 |

### Formulation Example 5. Screening of Sugar Concentrations

Formulations comprising 100 mg/mL of protein, 0.1 mg/mL of EDTA-2Na, 0.4 mg/mL of PS80, and sucrose at different concentrations as shown in Table 23 were prepared with 18 mM His-HCl buffer (pH 5.0). Forced degradation studies were performed on the samples (storage at 40°C for 4 weeks), with SEC, NR-CE, and IEC as evaluation indicators to investigate the effects of different sucrose concentrations on formulation stability.

The results are as shown in Table 23. ΔSEC ranged from 0.7% to 1.5%, and the formulation comprising 80 mg/mL of sucrose showed slightly better monomer purity than the other groups. No significant differences in ΔNR-CE and ΔIEC were observed among the groups.

**Table 23. Results of sucrose concentration screening**

| Group | Sucrose concentration (mg/mL) | Storage condition | ΔSEC% | ΔNR-CE% | ΔIEC% |
|---|---|---|---|---|---|
| 1 | 30 | 40°C W4 | 1.5 | 1.6 | 24.4 |
| 2 | 80 | 40°C W4 | 0.7 | 1.7 | 24.5 |
| 3 | 100 | 40°C W4 | 1.3 | 1.7 | 24.6 |

Sugar is a common excipient for biopharmaceuticals. Sucrose was used as an osmotic pressure regulator and stabilizer for formulation screening in this example, which can meet the formulation requirements. When the sucrose concentration was 80 mg/mL, the actual measured osmotic pressure of the formulation was 292 mOsm, which was close to isotonicity. Therefore, a sucrose concentration of 80 mg/mL is preferred.

### Formulation Example 6. Screening of Surfactant Types

Formulations comprising 70 mg/mL of protein, 80 mg/mL of sucrose, and different types of surfactants as shown in Table 24, including PS80 and Poloxamer 188 (P188), were prepared with 18 mM His-HCl buffer (pH 5.5). Forced degradation studies were performed on the samples (storage at 40°C for 4 weeks), with appearance, SEC, NR-CE, and iCIEF as evaluation indicators to investigate the effects of different surfactant types on protein stability.

The results are as shown in Table 24. The data of appearance, ΔSEC, ΔNR-CE, and ΔiCIEF showed that after storage at 40°C for 4 weeks, no significant differences in appearance and purity were observed between the formulations comprising different types of surfactants.

**Table 24. Results of surfactant concentration screening**

| Group | Surfactant type and concentration (mg/mL) | Storage condition | Appearance | ΔSEC% | ΔNR-CE% | ΔiCIEF% |
|---|---|---|---|---|---|---|
| 1 | PS80 (0.4 mg/mL) | 40°C W4 | Clear | 1.2 | 4.9 | 26.8 |
| 2 | P188 (2 mg/mL) | 40°C W4 | Clear | 1.1 | 3.8 | 27.8 |

### Formulation Example 7. Screening of Surfactant Concentrations

Formulations comprising 100 mg/mL of protein, 0.1 mg/mL of EDTA-2Na, 80 mg/mL of sucrose, and PS80 at different concentrations as shown in Table 25 were prepared with 18 mM His-HCl buffer (pH 5.0). Forced degradation studies were performed on the samples (storage at 40°C for 4 weeks), with appearance, SEC, NR-CE, and IEC as evaluation indicators to investigate the effects of different surfactant concentrations on protein stability.

The results are as shown in Table 25. The data of appearance, ΔSEC, ΔNR-CE, and ΔIEC showed that after storage at 40°C for 4 weeks, no significant differences in appearance and purity were observed among the formulations comprising different concentrations of PS80.

**Table 25. Results of surfactant concentration screening**

| Group | PS80 concentration (mg/mL) | Storage condition | Appearance | ΔSEC% | ΔNR-CE% | ΔIEC% |
|---|---|---|---|---|---|---|
| 1 | 0.1 | 40°C W4 | Clear | 0.9 | 1.4 | 24.0 |
| 2 | 0.4 | 40°C W4 | Clear | 0.7 | 1.7 | 24.5 |
| 3 | 1.0 | 40°C W4 | Clear | 1.0 | 1.5 | 23.8 |

### Formulation Example 8. Formulation Confirmation

A formulation comprising 150 mg/mL of protein, 0.1 mg/mL of EDTA-2Na, 80 mg/mL of sucrose, and 0.4 mg/mL of PS80 was prepared with 18 mM His-HCl buffer (pH 5.0). Long-term stability studies were performed on the sample (storage at 5°C for 6 months), with SEC and IEC as evaluation indicators to investigate the protein stability of this formulation.

The results are as shown in Table 26. After storage at 5°C for 6 months, no significant changes were observed in SEC and IEC as compared to Day 0, indicating good protein stability at this protein concentration.

**Table 26. Long-term stability results of the formulation at a protein concentration of 150 mg/mL**

| Group | Storage condition | SEC% | IEC% |
|---|---|---|---|
| | | Monomer | Neutral peak |
| 1 | Day 0 | 99.2 | 63.6 |
| 2 | 5°C, M6 | 98.6 | 62.8 |

### Formulation Example 9. Formulation Confirmation

A formulation comprising 100 mg/mL of protein, 0.1 mg/mL of EDTA-2Na, 0.4 mg/mL of PS80, and 80 mg/mL of sucrose was prepared with 18 mM His-HCl buffer (pH 5.0). Long-term stability studies were performed on the sample, with SEC, NR-CE, and IEC as evaluation indicators to investigate the long-term stability.

The results are as shown in Table 27. The data of SEC, NR-CE, and IEC showed that after storage at 5°C for 12 months, no significant changes in formulation purity were observed.

**Table 27. Long-term stability results**

| Group | Storage condition | SEC% | NR-CE% | IEC% |
|---|---|---|---|---|
| | | Monomer | Monomer | Neutral peak |
| | Day 0 | 98.4 | 99.3 | 61.3 |
| 1 | 5°C, M3 | 97.9 | 99.2 | 61.5 |
| | 5°C, M12 | 96.2 | 99.1 | 57.7 |

| | | | | |
|---|---|---|---|---|
| Notes: 5°C, M3/M12: storage at 5°C for 3 months/12 months. | | | | |

### Formulation Example 10. Formulation Confirmation

A formulation comprising 5 mg/mL of protein, 0.1 mg/mL of EDTA-2Na, 0.4 mg/mL of PS80, and 80 mg/mL of sucrose was prepared with 18 mM His-HCl buffer (pH 5.0). Long-term stability studies were performed on the sample, with SEC and IEC as evaluation indicators to investigate the long-term stability.

The results are as shown in Table 28. The data of SEC and IEC showed that after storage at 5°C for 6 months, no significant changes in formulation purity were observed.

**Table 28. Long-term stability results**

| Group | Storage condition | SEC% | IEC% |
|---|---|---|---|
| | | Monomer | Neutral peak |
| | Day 0 | 99.6 | 66.5 |
| 1 | 5°C, M1 | 99.6 | 66.7 |
| | 5°C, M6 | 99.7 | 63.8 |

| | | | |
|---|---|---|---|
| Notes: 5°C, M1/M6: storage at 5°C for 1 month/6 months. | | | |

Although the foregoing invention has been described in detail by way of drawings and examples for purposes of clarity of understanding, the descriptions and examples should not be construed as limiting the scope of the present invention. The disclosures of all the patents and scientific literature cited herein are clearly incorporated by reference in their entirety.

## Claims

1. A pharmaceutical composition, comprising a bispecific antibody specifically binding to DLL3 and CD3 and a buffer, wherein:
the bispecific antibody specifically binding to DLL3 and CD3 comprises a first chain having the structure as shown in Formula I and a second chain having the structure as shown in Formula II,
Formula I: [DLL3-VL]-[Linker 1]-[CD3-VH]-[Linker 2]-[Fc1],
Formula II: [CD3-VL]-[Linker 3]-[DLL3-VH]-[Linker 2]-[Fc2],
the DLL3-VH has a DLL3-HCDR1 comprising the amino acid sequence of SEQ ID NO: 14, a DLL3-HCDR2 comprising the amino acid sequence of SEQ ID NO: 15, and a DLL3-HCDR3 comprising the amino acid sequence of SEQ ID NO: 16; and the DLL3-VL has a DLL3-LCDR1 comprising the amino acid sequence of SEQ ID NO: 17, a DLL3-LCDR2 comprising the amino acid sequence of SEQ ID NO: 50, and a DLL3-LCDR3 comprising the amino acid sequence of SEQ ID NO: 19; and
the CD3-VH has a CD3-HCDR1 comprising the amino acid sequence of SEQ ID NO: 56, a CD3-HCDR2 comprising the amino acid sequence of SEQ ID NO: 57, and a CD3-HCDR3 comprising the amino acid sequence of SEQ ID NO: 58; and the CD3-VL has a CD3-LCDR1 comprising the amino acid sequence of SEQ ID NO: 59, a CD3-LCDR2 comprising the amino acid sequence of SEQ ID NO: 60, and a CD3-LCDR3 comprising the amino acid sequence of SEQ ID NO: 61;
wherein: the structures as shown in Formula I and Formula II are arranged from the N-terminus to the C-terminus; the Linker 1, Linker 2, and Linker 3 are the same or different peptide linkers; the Fc1 and the Fc2 are Fc region structural sequences capable of associating with each other via the Knob-into-Hole technique; and
the buffer is a histidine buffer, an acetate buffer, a citrate buffer, a succinate buffer, or a phosphate buffer;
preferably, the buffer is a histidine-histidine hydrochloride buffer, a histidine-histidine acetate buffer, or a citric acid-sodium citrate buffer; and
more preferably, the buffer is a histidine-histidine hydrochloride buffer.

2. The pharmaceutical composition according to claim 1, wherein the pharmaceutical composition has a pH of 4.5 to 6.0; preferably, the pharmaceutical composition has a pH of 4.5 to 5.5; more preferably, the pharmaceutical composition has a pH of 4.8 to 5.2; and most preferably, the pharmaceutical composition has a pH of about 5.0.

3. The pharmaceutical composition according to claim 1 or 2, wherein the bispecific antibody specifically binding to DLL3 and CD3 is at a concentration of 1 mg/mL to 250 mg/mL;
preferably, the bispecific antibody specifically binding to DLL3 and CD3 is at a concentration of 1 mg/mL to 150 mg/mL;
further preferably, the bispecific antibody specifically binding to DLL3 and CD3 is at a concentration of 70 mg/mL to 150 mg/mL;
more preferably, the bispecific antibody specifically binding to DLL3 and CD3 is at a concentration of 80 mg/mL to 120 mg/mL; and
most preferably, the bispecific antibody specifically binding to DLL3 and CD3 is at a concentration of about 100 mg/mL.

4. The pharmaceutical composition according to claim 1 or 2, wherein the bispecific antibody specifically binding to DLL3 and CD3 is at a concentration of 1 mg/mL to 20 mg/mL;
preferably, the bispecific antibody specifically binding to DLL3 and CD3 is at a concentration of 1 mg/mL to 10 mg/mL;
more preferably, the bispecific antibody specifically binding to DLL3 and CD3 is at a concentration of 4 mg/mL to 6 mg/mL; and
most preferably, the bispecific antibody specifically binding to DLL3 and CD3 is at a concentration of about 5 mg/mL.

5. The pharmaceutical composition according to any one of claims 1 to 4, wherein the pharmaceutical composition comprises a surfactant; preferably, the surfactant is a polysorbate or a poloxamer; more preferably, the surfactant is Polysorbate 80 or Poloxamer 188; and most preferably, the surfactant is Polysorbate 80.

6. The pharmaceutical composition according to claim 5, wherein the Polysorbate 80 is at a concentration of 0.01 mg/mL to 1.0 mg/mL; preferably, the Polysorbate 80 is at a concentration of 0.1 mg/mL to 1.0 mg/mL; further preferably, the Polysorbate 80 is at a concentration of 0.2 mg/mL to 0.6 mg/mL; more preferably, the Polysorbate 80 is at a concentration of 0.3 mg/mL to 0.5 mg/mL; and most preferably, the Polysorbate 80 is at a concentration of about 0.4 mg/mL.

7. The pharmaceutical composition according to claim 5, wherein the Poloxamer 188 is at a concentration of 0.5 mg/mL to 5.0 mg/mL; preferably, the Poloxamer 188 is at a concentration of 1 mg/mL to 3 mg/mL; more preferably, the Poloxamer 188 is at a concentration of 1.5 mg/mL to 2.5 mg/mL; and most preferably, the Poloxamer 188 is at a concentration of about 2.0 mg/mL.

8. The pharmaceutical composition according to any one of claims 1 to 7, wherein the pharmaceutical composition comprises a sugar; preferably, the sugar is sucrose, trehalose, mannitol, or sorbitol; and more preferably, the sugar is sucrose.

9. The pharmaceutical composition according to claim 8, wherein the sugar is at a concentration of 10 mg/mL to 120 mg/mL; preferably, the sugar is at a concentration of 30 mg/mL to 100 mg/mL; more preferably, the sugar is at a concentration of 64 mg/mL to 96 mg/mL; and most preferably, the sugar is at a concentration of about 80 mg/mL.

10. The pharmaceutical composition according to any one of claims 1 to 9, wherein the buffer is at a concentration of 5 mM to 100 mM; preferably, the buffer is at a concentration of 10 mM to 50 mM; further preferably, the buffer is at a concentration of 10 mM to 30 mM; more preferably, the buffer is at a concentration of 15 mM to 25 mM; and most preferably, the buffer is at a concentration of about 18 mM.

11. The pharmaceutical composition according to any one of claims 1 to 10, wherein the pharmaceutical composition further comprises an excipient; preferably, the excipient is disodium ethylenediaminetetraacetate dihydrate, DTPA, arginine hydrochloride, glycine, methionine, proline, histidine, phenylalanine, glutamic acid, aspartic acid, sodium chloride, or calcium chloride; and more preferably, the excipient is disodium ethylenediaminetetraacetate dihydrate.

12. The pharmaceutical composition according to claim 11, wherein the excipient is at a concentration of 0.01 mg/mL to 1 mg/mL; preferably, the excipient is at a concentration of 0.01 mg/mL to 0.5 mg/mL; more preferably, the excipient is at a concentration of 0.08 mg/mL to 0.12 mg/mL; and most preferably, the excipient is at a concentration of about 0.1 mg/mL.

13. The pharmaceutical composition according to any one of claims 1 to 12, wherein in the bispecific antibody specifically binding to DLL3 and CD3,
the DLL3-VH comprises the amino acid sequence of SEQ ID NO: 33, and the DLL3-VL comprises the amino acid sequence of SEQ ID NO: 42; and
the CD3-VH comprises the amino acid sequence of SEQ ID NO: 62, and the CD3-VL comprises the amino acid sequence of SEQ ID NO: 63.

14. The pharmaceutical composition according to any one of claims 1 to 13, wherein in the bispecific antibody specifically binding to DLL3 and CD3, the Fc1 has a knob in accordance with the Knob-into-Hole technique, and the Fc2 has a hole in accordance with the Knob-into-Hole technique;
preferably, the amino acid sequence of the Fc1 is as set forth in SEQ ID NO: 66; and the amino acid sequence of the Fc2 is as set forth in SEQ ID NO: 67.

15. The pharmaceutical composition according to any one of claims 1 to 14, wherein in the bispecific antibody specifically binding to DLL3 and CD3, the Linker 1, Linker 2, and Linker 3 are the same or different, and are each independently selected from the group consisting of SEQ ID NO: 68, SEQ ID NO: 69, and SEQ ID NO: 70.

16. The pharmaceutical composition according to any one of claims 1 to 15, wherein in the bispecific antibody specifically binding to DLL3 and CD3,
the amino acid sequence of Formula I is as set forth in SEQ ID NO: 71, and the amino acid sequence of Formula II is as set forth in SEQ ID NO: 72.

17. The pharmaceutical composition according to claim 1, wherein the pharmaceutical composition comprises the following components:
(a) 1 mg/mL to 250 mg/mL of the bispecific antibody specifically binding to DLL3 and CD3,
(b) 0.01 mg/mL to 1.0 mg/mL of Polysorbate 80 or 0.5 mg/mL to 5 mg/mL of Poloxamer 188,
(c) 10 mg/mL to 120 mg/mL of a sugar,
(d) 0.01 mg/mL to 1 mg/mL of disodium ethylenediaminetetraacetate dihydrate, and
(e) 5 mM to 100 mM of a buffer, the pharmaceutical composition having a pH of 4.5 to 6.0; preferably, the pharmaceutical composition comprises the following components:
(a) 1 mg/mL to 150 mg/mL of the bispecific antibody specifically binding to DLL3 and CD3,
(b) 0.1 mg/mL to 1.0 mg/mL of Polysorbate 80 or 1 mg/mL to 3 mg/mL of Poloxamer 188,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, a histidine-histidine acetate buffer, or a citric acid-sodium citrate buffer, the pharmaceutical composition having a pH of 4.5 to 5.5;
further preferably, the pharmaceutical composition comprises the following components:
(a) 70 mg/mL to 150 mg/mL of the bispecific antibody specifically binding to DLL3 and CD3,
(b) 0.2 mg/mL to 0.6 mg/mL of Polysorbate 80 or 1 mg/mL to 3 mg/mL of Poloxamer 188,
(c) 64 mg/mL to 96 mg/mL of sucrose,
(d) 0.01 mg/mL to 0.5 mg/mL of disodium ethylenediaminetetraacetate dihydrate, and
(e) 10 mM to 30 mM of a histidine-histidine hydrochloride buffer, the pharmaceutical composition having a pH of 4.5 to 5.5;
more preferably, the pharmaceutical composition comprises the following components:
(a) 80 mg/mL to 120 mg/mL of the bispecific antibody specifically binding to DLL3 and CD3,
(b) 0.3 mg/mL to 0.5 mg/mL of Polysorbate 80 or 1.5 mg/mL to 2.5 mg/mL of Poloxamer 188,
(c) 64 mg/mL to 96 mg/mL of sucrose,
(d) 0.08 mg/mL to 0.12 mg/mL of disodium ethylenediaminetetraacetate dihydrate, and
(e) 15 mM to 25 mM of a histidine-histidine hydrochloride buffer, the pharmaceutical composition having a pH of 4.8 to 5.2; and
most preferably, the pharmaceutical composition comprises the following components:
(a) about 100 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of about 5.0.

18. The pharmaceutical composition according to claim 1, wherein the pharmaceutical composition comprises the following components:
(a) 1 mg/mL to 20 mg/mL of the bispecific antibody specifically binding to DLL3 and CD3,
(b) 0.01 mg/mL to 1.0 mg/mL of Polysorbate 80 or 0.5 mg/mL to 5 mg/mL of Poloxamer 188,
(c) 10 mg/mL to 120 mg/mL of a sugar,
(d) 0.01 mg/mL to 1 mg/mL of disodium ethylenediaminetetraacetate dihydrate, and
(e) 5 mM to 100 mM of a buffer, the pharmaceutical composition having a pH of 4.5 to 6.0;
preferably, the pharmaceutical composition comprises the following components:
(a) 1 mg/mL to 10 mg/mL of the bispecific antibody specifically binding to DLL3 and CD3,
(b) 0.1 mg/mL to 1.0 mg/mL of Polysorbate 80 or 0.5 mg/mL to 5 mg/mL of Poloxamer 188,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, a histidine-histidine acetate buffer, or a citric acid-sodium citrate buffer, the pharmaceutical composition having a pH of 4.5 to 5.5;
further preferably, the pharmaceutical composition comprises the following components:
(a) 1 mg/mL to 10 mg/mL of the bispecific antibody specifically binding to DLL3 and CD3,
(b) 0.1 mg/mL to 1.0 mg/mL of Polysorbate 80,
(c) 30 mg/mL to 100 mg/mL of sucrose,
(d) 0.01 mg/mL to 1 mg/mL of disodium ethylenediaminetetraacetate dihydrate, and
(e) 10 mM to 50 mM of a histidine-histidine hydrochloride buffer, the pharmaceutical composition having a pH of 4.5 to 5.5;
more preferably, the pharmaceutical composition comprises the following components:
(a) 4 mg/mL to 6 mg/mL of the bispecific antibody specifically binding to DLL3 and CD3,
(b) 0.3 mg/mL to 0.5 mg/mL of Polysorbate 80,
(c) 64 mg/mL to 96 mg/mL of sucrose,
(d) 0.08 mg/mL to 0.12 mg/mL of disodium ethylenediaminetetraacetate dihydrate, and
(e) 15 mM to 25 mM of a histidine-histidine hydrochloride buffer, the pharmaceutical composition having a pH of 4.8 to 5.2;
most preferably, the pharmaceutical composition comprises the following components:
(a) about 5 mg/mL of the bispecific antibody specifically binding to DLL3 and CD3,
(b) about 0.4 mg/mL of Polysorbate 80,
(c) about 80 mg/mL of sucrose,
(d) about 0.1 mg/mL of disodium ethylenediaminetetraacetate dihydrate, and
(e) about 18 mM of a histidine-histidine hydrochloride buffer, the pharmaceutical composition having a pH of about 5.0.

19. The pharmaceutical composition according to any one of claims 1 to 18, which is a subcutaneous injection formulation, an intravenous injection formulation, an intraperitoneal injection formulation, or an intramuscular injection formulation, preferably a subcutaneous injection formulation.

20. A method for preparing a lyophilized formulation, comprising the step of lyophilizing the pharmaceutical composition according to any one of claims 1 to 18.

21. A lyophilized formulation obtained by the method according to claim 20.

22. Use of the pharmaceutical composition according to any one of claims 1 to 19 or the lyophilized formulation according to claim 21 in the manufacture of a medicament for the treatment of a tumor or cancer, preferably wherein the tumor or cancer is selected from the group consisting of:
lung cancer, small cell lung cancer, large cell lung cancer, squamous cell carcinoma of the head and neck, head and neck cancer, brain cancer, glioma, glioblastoma multiforme, neuroblastoma, central nervous system cancer, neuroendocrine tumor, throat cancer, pharyngeal squamous cell carcinoma, oral squamous cell carcinoma, nasopharyngeal carcinoma, esophageal cancer, thyroid cancer, malignant pleural mesothelioma, breast cancer, liver cancer, hepatobiliary cancer, pancreatic cancer, gastric cancer, gastrointestinal cancer, intestinal cancer, colon cancer, colorectal cancer, kidney cancer, clear cell renal cell carcinoma, ovarian cancer, endometrial cancer, cervical cancer, bladder cancer, prostate cancer, testicular cancer, skin cancer, melanoma, large cell lung cancer, triple-negative breast cancer, and lymphoma.
